# EUROPÄISCHE PATENTANMELDUNG

(11) **EP 2 070 900 A2**
(43) Veröffentlichungstag der Anmeldung: **17.06.2009**
(21) Anmeldenummer: 09153296.0
(22) Anmeldetag: 16.07.2007
(51) Int. Cl.: C07C 29/09, C07C 31/10, C07C 31/12, C07C 5/333, C07C 11/06, C07C 11/08, C07C 67/04, C07C 69/06, C07C 69/14, C07C 51/09, C07C 53/02, C07C 53/08

(54) **Verfahren zur Herstellung von Isopropanol und 2-Butanol aus den entsprechenden Alkanen**

(30) Priorität: 20.07.2006 EP 06117568
(62) Teilanmeldung aus: 07787557.3
(71) Anmelder: BASF SE, 67056 Ludwigshafen (DE)
(72) Erfinder: Degen, Georg, 64653, Lorsch (DE); Crone, Sven, 67117, Ludwigshafen (DE); Boehling, Ralf, 64653, Lorsch (DE); Altenhoff, Ansgar Gereon, 69117, Heidelberg (DE); Rohde, Wolfgang, 67346, Speyer (DE); Buerkle, Jochen, 68163, Mannheim (DE); Schindler, Goetz-Peter, 67071, Ludwigshafen (DE); Holtmann, Thomas, 67346, Speyer (DE); Schmitt, Markus, 69115, Heidelberg (DE)
(74) Vertreter: Isenbruck, Günter

(57) **Zusammenfassung**

Verfahren zur Herstellung von Alkanolen (I), ausgewählt aus der Gruppe bestehend aus Isopropanol und 2-Butanol, aus den entsprechenden Alkanen (II), ausgewählt aus der Gruppe bestehend aus Propan und n-Butan, mit den Schritten:
A) ein das Alkan (II) enthaltender Einsatzgasstrom a wird bereitgestellt;
B) der das Alkan (II) enthaltende Einsatzgasstrom a wird in eine Dehydrierzo-ne eingespeist und das Alkan (II) wird einer Dehydrierung zum Alken (III) unterworfen, wobei ein Produktgasstrom b enthaltend das Alken (III), nicht umgesetztes Alkan (II), gegebenenfalls Hochsieder, Wasserdampf, Was-serstoff und Leichtsieder erhalten wird;
C) der Produktgasstrom b wird zumindest komprimiert, optional wird der Produktgasstrom b in eine wässrige Phase c1, eine das Alken (III), das Alkan (II) und gegebenenfalls Hochsieder enthaltende Phase c2 und eine Gasphase c3 enthaltend Wasserstoff und Leichtsieder aufgetrennt;
D) der Produktgasstrom b beziehungsweise die Alken (III) und Alkan (II) enthaltende Phase c2 wird in einer Esterbildungszone mit einer organischen Säure (IV) umgesetzt, wobei ein Produktgemisch d enthaltend den entsprechenden Alkylester (V) der organischen Säure und das nicht umgesetzte Alkan (II) erhalten wird;
E) von dem Produktgemisch d wird ein Alkan (II) enthaltender Gasstrom e1 abgetrennt, der gegebenenfalls in die Dehydrierzone zurückgeführt wird, und ein den Alkylester enthaltendes Produktgemisch e2 gewonnen;
F) das den Alkylester enthaltende Produktgemisch e2 wird in einer Esterspaltungszone mit Wasser zu einem Produktgemisch f enthaltend das Alkanol (I) und die organische Säure (IV) umgesetzt;
G) aus dem Produktgemisch f werden das Alkanol (I) und die organische Säure (IV) abgetrennt und die organische Säure wird gegebenenfalls in die Esterbildungszone zurückgeführt.

## Beschreibung

Die Erfindung betrifft ein Verfahren zur Herstellung von Isopropanol aus Propan und von 2-Butanol aus Butan.

Die Hydratisierung von Alkenen zu Alkoholen ist wohl bekannt und wird großtechnisch betrieben. Technisch ausgeübt wird die zweistufige Hydratisierung, bei der das Alken mit Schwefelsäure zum Alkylsulfat umgesetzt und dieses im zweiten Schritt mit Wasser zum Alkohol und der Säure gespalten wird. Vorteilhaft bei diesem Verfahren ist, dass das Alken in Form von Alken-Rohgemischen, beispielsweise Gemischen aus dem Alken, dem entsprechenden Alkan und weiteren Nebenbestandteilen, eingesetzt werden kann. Nachteilig ist das hoch korrosive Medium, die Verunreinigung des Produkts mit schwefelhaltigen geruchsbildenden Stoffen, welche zusätzliche Reinigungsschritte erforderlich machen kann, und der Verlust des inerten Alkananteils des Gemischs, welcher nicht reagiert und nach der Reaktion ausgeschleust wird. Nachteilig ist weiterhin, dass nach Hydrolyse des Alkylsulfats zum Alkohol die anfallende verdünnte Schwefelsäure vor ihrer Wiederverwendung in dem Esterbildungsschritt aufkonzentriert werden muss.

Daneben ist die Esterbildung von Olefinen mit Carbonsäuren und anschließende Esterspaltung der gebildeten Alkylester zum entsprechenden Alkohol unter Wiedergewinnung der Säure bekannt. So beschreibt US 4,384,148 die Umsetzung von Ethen mit Essigsäure zu Ethylacetat in einem Autoklaven. Die anschließende Hydrolyse des destillativ abgetrennten Ethylacetats mit Wasser im Molverhältnis 1 : 5 in einem Autoklaven liefert ein Gemisch enthaltend Ethylacetat, Ethanol und Diethylether. GB 2 238 539 offenbart die zweistufige Hydratisierung von 1-Buten durch Umsetzung mit Trifluoressigsäure zum 2-Butyltrifluoracetat in Gegenwart eines stark sauren lonenaustauscherharzes und anschließende Hydrolyse des Esters zum 2-Butanol.

Weiterhin wird technisch die direkte, einstufige Hydratisierung von Alkenen an stark sauren Katalysatoren, beispielsweise Ionentauschern, Zeolithen, Heteropolysäuren und Mineralsäuren, auf heterogenen Trägern durchgeführt. Verfahren zur Direkt-Hydratisierung können in der Gasphase, in der flüssigen Phase oder zweiphasig durchgeführt werden. Nachteilig an den einstufigen Verfahren sind insbesondere die niedrigen Umsätze und die hohen Anforderungen an die Reinheit des eingesetzten Alkens. Beispielsweise muss Propylen als Polymer Grade-Propylen eingesetzt werden.

GB-A 2 147 290 offenbart ein Verfahren zur Herstellung von Isopropanol, 2-Butanol und Methyl-tert.-butylether aus einem LPG-Gasgemisch enthaltend Propan, n-Butan und iso-Butan. Das Gasgemisch wird zu einem Gemisch enthaltend Propen, n-Butene und Isobuten dehydriert und passiert anschließend eine Veretherungszone, in der Isobuten mit Methanol zu Methyl-tert.-butylether verethert wird. Propen und n-Butene, welche unter den gewählten Bedingungen im Wesentlichen nicht mit Methanol reagieren, werden simultan mit Wasser zu Isopropanol beziehungsweise 2-Butanol direkt hydratisiert.

US 2004/0225165 A offenbart ein Verfahren zur Herstellung von Alkoholen mit 3 oder mehr C-Atomen aus den entsprechenden Alkanen, bei dem ein Strom enthaltend Propan oder ein längerkettiges Alkan zu einem Zwischenproduktstrom enthaltend das entsprechende Olefin umgesetzt wird, und der Zwischenproduktstrom durch direkte oder indirekte Hydratisierung zu einem Produktstrom enthaltend den entsprechenden Alkohol umgesetzt wird. Die Schrift nennt als indirekten Prozess die zweistufige Hydratisierung von Propen mit konzentrierter Schwefelsäure unter intermediärer Bildung des Schwefelsäureesters und anschließender Hydrolyse des Schwefelsäureesters zum Alkohol. Daneben werden verschiedene Verfahren der direkten Hydratisierung beschrieben.

Aus US 4,484,013 ist ein Verfahren zur Herstellung von Isopropanol und tert.-Butanol bekannt, bei dem ein Einsatzgasstrom aus Propan und Isobutan in eine Dehydrierungszone eingespeist und zu einem Produktgasgemisch enthaltend Propen, Isobuten, sowie nicht umgesetztes Propan und Isobutan nicht-oxidativ, d. h. in Abwesenheit von Luft oder Sauerstoff, dehydriert wird. Aus dem Produktgasstrom der Dehydrierung werden zunächst Leichtsieder (Wasserstoff) und anschließend Propan destillativ abgetrennt, wobei letzteres in die Dehydrierzone zurückgeführt wird. Der verbleibende Gasstrom, der im Wesentlichen aus Propen, Isobuten und Isobutan besteht, wird in eine Hydratisierungszone eingespeist, wo Propen und Isobuten an einem sauren lonentauscherharz direkt zu Isopropanol und tert.-Butanol hydratisiert werden. Der verbleibende Gasstrom wird einerseits in einen Isobutanstrom, der in die Dehydrierzone zurückgeführt wird, und andererseits in einen Propan und Propen enthaltenden Strom, der in die Propan-Abtrennung vor den Hydratisierungsschritt zurückgeführt wird, aufgetrennt. Das Verfahren ist somit dadurch gekennzeichnet, dass vor der Hydratisierung nicht umgesetztes Propan abgetrennt und die Hydratisierung mit einem im Wesentlichen aus den C₃- und C₄-Olefinen bestehenden Einsatzstrom durchgeführt wird.

Aufgabe der Erfindung ist es, ein wirtschaftliches Verfahren zur Herstellung von Isopropanol und 2-Butanol bereitzustellen, welches die Nachteile des Standes der Technik nicht aufweist.

Gelöst wird die Aufgabe durch ein Verfahren zur Herstellung von Alkanolen (I), ausgewählt aus der Gruppe bestehend aus Isopropanol und 2-Butanol, aus den entsprechenden Alkanen (II), ausgewählt aus der Gruppe bestehend aus Propan und n-Butan, mit den Schritten:
A) ein das Alkan (II) enthaltender Einsatzgasstrom a wird bereitgestellt;
B) der das Alkan (II) enthaltende Einsatzgasstrom a wird in eine Dehydrierzone eingespeist und das Alkan (II) wird einer Dehydrierung zum Alken (III) unterworfen, wobei ein Produktgasstrom b enthaltend das Alken (III), nicht umgesetztes Alkan (II), gegebenenfalls Hochsieder, Wasserdampf, Wasserstoff und Leichtsieder erhalten wird;
C) der Produktgasstrom b wird zumindest komprimiert, optional wird der Produktgasstrom b in eine wässrige Phase c1, eine das Alken (III), das Alkan (II) und gegebenenfalls Hochsieder enthaltende Phase c2 und eine Gasphase c3 enthaltend Wasserstoff und Leichtsieder aufgetrennt;
D) der Produktgasstrom b beziehungsweise die Alken (III) und Alkan (II) enthaltende Phase c2 wird in einer Esterbildungszone mit einer organischen Säure (IV) umgesetzt, wobei ein Produktgemisch d enthaltend den entsprechenden Alkylester (V) der organischen Säure und das nicht umgesetzte Alkan (II) erhalten wird;
E) von dem Produktgemisch d wird ein Alkan (II) enthaltender Gasstrom e1 abgetrennt, der gegebenenfalls in die Dehydrierzone zurückgeführt wird, und ein den Alkylester enthaltendes Produktgemisch e2 gewonnen;
F) das den Alkylester enthaltende Produktgemisch e2 wird in einer Esterspaltungszone mit Wasser zu einem Produktgemisch f enthaltend das Alkanol (I) und die organische Säure (IV) umgesetzt;
G) aus dem Produktgemisch f werden das Alkanol (I) und die organische Säure (IV) abgetrennt und die organische Säure gegebenenfalls in die Esterbildungszone zurückgeführt.

Das erfindungsgemäße Verfahren verzichtet auf den Einsatz von stark korrosiver Schwefelsäure. Es zeichnet sich dadurch aus, dass dennoch in dem Esterbildungsschritt D) auch bei Verwendung eines Einsatzgasstromes, der das Alken (I) nur in sehr verdünnter Form neben weiteren Komponenten (nicht umgesetztes Alkan, Inertgase) enthält, hohe Raum/Zeit-Ausbeuten und Umsätze erzielt werden. Auf eine Abtrennung von nicht reaktiven Nebenkomponenten vor Durchführung des Esterbildungsschrittes kann daher verzichtet werden. Da der Esterspaltungsschritt F) mit Wasser in stöchiometrischem Unterschuss durchgeführt werden kann, kann ferner die organische Säure (IV) in konzentrierter Form gewonnen und direkt, ohne weitere Aufkonzentrierung, in den Esterbildungsschritt D) zurückgeführt werden.

In dem ersten Verfahrensteil A) wird ein Alkan (II), ausgewählt aus Propan und Butan, enthaltender Einsatzgasstrom a bereitgestellt. Im Falle von Propan enthält dieser im Allgemeinen mindestens 80 Vol.-% Propan, vorzugsweise 90 Vol.-% Propan. Daneben enthält er im Allgemeinen noch Butane (n-Butan, iso-Butan), Butene, Ethan und Ethen. Typische Zusammensetzungen des propanhaltigen Einsatzgasstroms sind in DE-A 102 46 119 und DE-A 102 45 585 offenbart. Üblicherweise wird der propanhaltige Einsatzgasstrom a aus liquefied petroleum gas (LPG) gewonnen.

Im Falle von n-Butan als Alkan (II) enthält der Einsatzgasstrom im Allgemeinen mindestens 80 Vol.-% n-Butan, vorzugsweise 90 Vol.-% n-Butan. Daneben enthält er im Allgemeinen noch Ethan, Ethen, Propan, Propen, iso-Butan, Butene und C₅-Koh lenwasserstoffe.

In einem Verfahrensteil B) wird der das Alkan (II) enthaltende Einsatzgasstrom in eine Dehydrierzone eingespeist und einer im Allgemeinen katalytischen Dehydrierung unterworfen. Dabei wird das Alkan in einem Dehydrierreaktor an einem dehydrieraktiven Katalysator teilweise zum Alken dehydriert. Daneben fallen Wasserstoff und in geringen Mengen Leichtsieder sowie Hochsieder an. Als Leichtsieder werden vorliegend niedriger als Propen beziehungsweise 1-Buten siedende Kohlenwasserstoffe bezeichnet, als Schwersieder höher als Propen beziehungsweise 2-Buten siedende Kohlenwasserstoffe bezeichnet. So können beispielsweise bei der Propan-Dehydrierung als Leichtsieder Methan, Ethan und Ethen und als Hochsieder C₄⁺-Kohlenwasserstoffe (n-Butan, iso-Butan, Butene, Butadien) anfallen. Bei der n-Butan-Dehydrierung können beispielsweise als Leichtsieder Methan, Ethan und Ethen, Propan und Propen und als Hochsieder C₅⁺-Kohlenwasserstoffe anfallen. Außerdem fallen, falls die Dehydrierung in Gegenwart eines sauerstoffhaltigen Gases durchgeführt wird, im Allgemeinen Kohlenstoffoxide (CO, CO₂) insbesondere CO₂, sowie Wasserdampf und gegebenenfalls in geringem Umfang Inertgase im Produktgasgemisch der Dehydrierung an. Der Produktgasstrom der Dehydrierung enthält im Allgemeinen Wasserdampf, der bereits dem Dehydriergasgemisch zugesetzt und/oder - bei Dehydrierung in Gegenwart von Sauerstoff (oxidativ oder nicht-oxidativ) - bei der Dehydrierung gebildet wird. Inertgase (Stickstoff) werden bei Durchführung der Dehydrierung in Gegenwart von Sauerstoff mit dem eingespeisten sauerstoffhaltigen Gasstrom in die Dehydrierzone eingebracht, sofern kein reiner Sauerstoff eingespeist wird. Daneben liegt im Reaktionsgasgemisch nicht umgesetztes Alkan (II) (Propan beziehungsweise n-Butan) vor.

Die Alkan-Dehydrierung kann grundsätzlich in allen aus dem Stand der Technik bekannten Reaktortypen durchgeführt werden. Eine vergleichsweise ausführliche Beschreibung von erfindungsgemäß geeigneten Reaktortypen enthält auch "Catalytica^{®} Studies Division, Oxidative Dehydrogenation and Alternative Dehydrogenation Processes" (Study Number 4192 OD, 1993, 430 Ferguson Drive, Mountain View, California, 94043-5272, USA).

Die Dehydrierung kann als oxidative oder nicht-oxidative Dehydrierung durchgeführt werden. Die Dehydrierung kann isotherm oder adiabat durchgeführt werden. Die Dehydrierung kann katalytisch im Festbett-, Wanderbett- oder Wirbelbettreaktor durchgeführt werden.

Die nicht-oxidative katalytische Alkan-Dehydrierung wird bevorzugt autotherm durchgeführt. Dazu wird dem Reaktionsgasgemisch der Dehydrierung in mindestens einer Reaktionszone zusätzlich Sauerstoff zugemischt und der in dem Reaktionsgasgemisch enthaltene Wasserstoff und/oder Kohlenwasserstoff zumindest teilweise verbrannt, wodurch zumindest ein Teil der benötigten Dehydrierwärme in der mindestens einen Reaktionszone direkt in dem Reaktionsgasgemisch erzeugt wird.

Ein Merkmal der nicht-oxidativen Fahrweise gegenüber einer oxidativen Fahrweise ist die zumindest intermediäre Bildung von Wasserstoff, die sich im Vorhandensein von Wasserstoff im Produktgas der Dehydrierung niederschlägt. Bei der oxidativen Dehydrierung findet sich kein freier Wasserstoff im Produktgas der Dehydrierung.

Eine geeignete Reaktorform ist der Festbettrohr- oder Rohrbündelreaktor. Bei diesen befindet sich der Katalysator (Dehydrierungskatalysator und gegebenenfalls spezieller Oxidationskatalysator) als Festbett in einem Reaktionsrohr oder in einem Bündel von Reaktionsrohren. Übliche Reaktionsrohr-Innendurchmesser betragen etwa 10 bis 15 cm. Ein typischer Dehydrierrohrbündelreaktor umfasst ca. 300 bis 1000 Reaktionsrohre. Die Temperatur im Reaktionsrohrinneren bewegt sich üblicherweise im Bereich von 300 bis 1200°C, vorzugsweise im Bereich von 500 bis 1000°C. Der Arbeitsdruck liegt üblicherweise zwischen 0,5 und 8 bar, häufig zwischen 1 und 2 bar bei Verwendung einer geringen Wasserdampfverdünnung, aber auch zwischen 3 und 8 bar bei Verwendung einer hohen Wasserdampfverdünnung (entsprechend dem so genannten "steam active reforming process" (STAR-Prozess) oder dem Linde-Verfahren) zur Dehydrierung von Propan oder Butan von Phillips Petroleum Co. Typische Katalysatorbelastungen (GHSV) liegen bei 500 bis 2000 h⁻¹, bezogen auf eingesetzten Kohlenwasserstoff. Die Katalysatorgeometrie kann beispielsweise kugelförmig oder zylindrisch (hohl oder voll) sein. Es können auch mehrere Festbettrohr- oder Rohrbündelreaktoren nebeneinander betrieben werden, von denen sich abwechselnd mindestens einer im Zustand der Regenerierung befindet.

Die nicht-oxidative katalytische, autotherme Dehydrierung kann auch, entsprechend dem Snamprogetti/Yarsintez-FBD-Prozess, heterogen katalysiert im Wirbelbett durchgeführt werden. Zweckmäßigerweise werden dabei zwei Wirbelbetten nebeneinander betrieben, von denen sich eines in der Regel im Zustand der Regenerierung befindet. Der Arbeitsdruck beträgt typischerweise 1 bis 2 bar, die Dehydriertemperatur in der Regel 550 bis 600°C. Die für die Dehydrierung erforderliche Wärme kann dabei in das Reaktionssystem eingebracht werden, indem der Dehydrierkatalysator auf die Reaktionstemperatur vorerhitzt wird. Durch die Zumischung eines Sauerstoff enthaltenden Co-Feeds kann auf die Vorerhitzer verzichtet werden, und die benötigte Wärme wird direkt im Reaktorsystem durch Verbrennung von Wasserstoff und/oder Kohlenwasserstoffen in Gegenwart von Sauerstoff erzeugt. Gegebenenfalls kann zusätzlich ein Wasserstoff enthaltender Co-Feed zugemischt werden.

Die nicht-oxidative katalytische, autotherme Dehydrierung wird bevorzugt in einem Hordenreaktor durchgeführt. Dieser enthält ein oder mehrere aufeinander folgende Katalysatorbetten. Die Anzahl der Katalysatorbetten kann 1 bis 20, zweckmäßigerweise 1 bis 6, bevorzugt 1 bis 4 und insbesondere 1 bis 3 betragen. Die Katalysatorbetten werden vorzugsweise radial oder axial vom Reaktionsgas durchströmt. Im Allgemeinen wird ein solcher Hordenreaktor mit einem Katalysatorfestbett betrieben. Im einfachsten Fall sind die Katalysatorfestbetten in einem Schachtofenreaktor axial oder in den Ringspalten von konzentrisch angeordneten zylindrischen Gitterrosten angeordnet. Ein Schachtofenreaktor entspricht einer Horde. Die Durchführung der Dehydrierung in einem einzelnen Schachtofenreaktor entspricht einer Ausführungsform. In einer weiteren bevorzugten Ausführungsform wird die Dehydrierung in einem Hordenreaktor mit 3 Katalysatorbetten durchgeführt.

Im Allgemeinen wird die Menge des dem Reaktionsgasgemisch zugesetzten sauerstoffhaltigen Gases so gewählt, dass durch die Verbrennung von im Reaktionsgasgemisch vorhandenem Wasserstoff und gegebenenfalls von im Reaktionsgasgemisch vorliegenden Kohlenwasserstoffen und/oder von in Form von Koks vorliegendem Kohlenstoff die für die Dehydrierung des Alkans (Propan beziehungsweise n-Butan) benötigte Wärmemenge erzeugt wird. Im Allgemeinen beträgt die insgesamt zugeführte Sauerstoffmenge, bezogen auf die Gesamtmenge des Propans, 0,001 bis 0,5 mol/mol, bevorzugt 0,005 bis 0,25 mol/mol, besonders bevorzugt 0,05 bis 0,25 mol/mol. Sauerstoff kann entweder als reiner Sauerstoff oder als sauerstoffhaltiges Gas, welches Inertgase enthält, eingesetzt werden. Um hohe Propan- und Propen-Verluste bei der Aufarbeitung (siehe unten) zu vermeiden, kann es vorteilhaft sein, wenn der Sauerstoff-Gehalt des eingesetzten sauerstoffhaltigen Gases hoch ist und mindestens 50 Vol.-%, bevorzugt mindestens 80 Vol.-%, besonders bevorzugt mindestens 90 Vol.-% beträgt. Besonders vorteilhaft kann es sein, wenn das sauerstoffhaltige Gas technisch reiner Sauerstoff mit einem O₂-Gehalt von ca. 99 Vol.-% ist. Daneben ist eine Fahrweise möglich, bei der Luft als sauerstoffhaltiges Gas eingespeist wird.

Der zur Wärmeerzeugung verbrannte Wasserstoff ist der bei der katalytischen Alkan-Dehydrierung gebildete Wasserstoff sowie gegebenenfalls dem Reaktionsgasgemisch als wasserstoffhaltiges Gas zusätzlich zugesetzter Wasserstoff. Vorzugsweise sollte soviel Wasserstoff zugegen sein, dass das Molverhältnis H₂/O₂ im Reaktionsgasgemisch unmittelbar nach der Einspeisung von Sauerstoff 1 bis 10, bevorzugt 2 bis 5 mol/mol beträgt. Dies gilt bei mehrstufigen Reaktoren für jede Zwischeneinspeisung von sauerstoffhaltigem und gegebenenfalls wasserstoffhaltigem Gas.

Die Wasserstoffverbrennung erfolgt katalytisch. Der eingesetzte Dehydrierungskatalysator katalysiert im Allgemeinen auch die Verbrennung der Kohlenwasserstoffe und von Wasserstoff mit Sauerstoff, so dass grundsätzlich kein von diesem verschiedener, spezieller Oxidationskatalysator erforderlich ist. In einer Ausführungsform wird in Gegenwart eines oder mehrerer Oxidationskatalysatoren gearbeitet, die selektiv die Verbrennung von Wasserstoff zu Sauerstoff in Gegenwart von Kohlenwasserstoffen katalysieren. Die Verbrennung dieser Kohlenwasserstoffe mit Sauerstoff zu CO, CO₂ und Wasser läuft dadurch nur in untergeordnetem Maße ab. Vorzugsweise liegen der Dehydrierungskatalysator und der Oxidationskatalysator in verschiedenen Reaktionszonen vor.

Bei mehrstufiger Reaktionsführung kann der Oxidationskatalysator in nur einer, in mehreren oder in allen Reaktionszonen vorliegen.

Bevorzugt ist der Katalysator, der selektiv die Oxidation von Wasserstoff katalysiert, an den Stellen angeordnet, an denen höhere Sauerstoffpartialdrucke herrschen als an anderen Stellen des Reaktors, insbesondere in der Nähe der Einspeisungsstelle für das sauerstoffhaltige Gas. Die Einspeisung von sauerstoffhaltigem Gas und/oder wasserstoffhaltigem Gas kann an einer oder mehreren Stellen des Reaktors erfolgen.

In einer Ausführungsform des erfindungsgemäßen Verfahrens erfolgt eine Zwischeneinspeisung von sauerstoffhaltigem Gas und von wasserstoffhaltigem Gas vor jeder Horde eines Hordenreaktors. In einer weiteren Ausführungsform des erfindungsgemäßen Verfahrens erfolgt die Einspeisung von sauerstoffhaltigem Gas und von wasserstoffhaltigem Gas vor jeder Horde außer der ersten Horde. In einer Ausführungsform ist hinter jeder Einspeisungsstelle eine Schicht aus einem speziellen Oxidationskatalysator vorhanden, gefolgt von einer Schicht aus dem Dehydrierungskatalysator. In einer weiteren Ausführungsform ist kein spezieller Oxidationskatalysator vorhanden. Die Dehydriertemperatur beträgt im allgemeinen 400 bis 1100°C, der Druck im letzten Katalysatorbett des Hordenreaktors im Allgemeinen 0,2 bis 5 bar, bevorzugt 1 bis 3 bar. Die Belastung (GHSV = gasous hourly space velocity) beträgt im Allgemeinen 500 bis 2000 h⁻¹, bei Hochlastfahrweise auch bis zu 100 000 h⁻¹, bevorzugt 4000 bis 16 000 h⁻¹.

Ein bevorzugter Katalysator, der selektiv die Verbrennung von Wasserstoff katalysiert, enthält Oxide und/oder Phosphate, ausgewählt aus der Gruppe bestehend aus den Oxiden und/oder Phosphaten von Germanium, Zinn, Blei, Arsen, Antimon oder Bismut. Ein weiterer bevorzugter Katalysator, der die Verbrennung von Wasserstoff katalysiert, enthält ein Edelmetall der VIII. und/oder I. Nebengruppe.

Die eingesetzten Dehydrierungskatalysatoren weisen im Allgemeinen einen Träger und eine Aktivmasse auf. Der Träger besteht dabei in der Regel aus einem wärmebeständigen Oxid oder Mischoxid. Bevorzugt enthalten die Dehydrierungskatalysatoren ein Metalloxid, das ausgewählt ist aus der Gruppe bestehend aus Zirkondioxid, Zinkoxid, Aluminiumoxid, Siliciumdioxid, Titandioxid, Magnesiumoxid, Lanthanoxid, Ceroxid und deren Gemischen, als Träger. Bei den Gemischen kann es sich um physikalische Mischungen oder auch um chemische Mischphasen wie Magnesium- oder Zinkaluminiumoxid-Mischoxide handeln. Bevorzugte Träger sind Zirkondioxid und/oder Siliziumdioxid, besonders bevorzugt sind Gemische aus Zirkondioxid und Siliziumdioxid.

Die Aktivmasse der Dehydrierungskatalysatoren enthält im Allgemeinen ein oder mehrere Elemente der VIII. Nebengruppe, bevorzugt Platin und/oder Palladium, besonders bevorzugt Platin. Darüber hinaus können die Dehydrierungskatalysatoren ein oder mehrere Elemente der I. und/oder II. Hauptgruppe aufweisen, bevorzugt Kalium und/oder Cäsium. Weiterhin können die Dehydrierungskatalysatoren ein oder mehrere Elemente der III. Nebengruppe einschließlich der Lanthaniden und Actiniden enthalten, bevorzugt Lanthan und/oder Cer. Schließlich können die Dehydrierungskatalysatoren ein oder mehrere Elemente der III. und/oder IV. Hauptgruppe aufweisen, bevorzugt ein oder mehrere Elemente aus der Gruppe bestehend aus Bor, Gallium, Silizium, Germanium, Zinn und Blei, besonders bevorzugt Zinn.

In einer bevorzugten Ausführungsform enthält der Dehydrierungskatalysator mindestens ein Element der VIII. Nebengruppe, mindestens ein Element der I. und/oder II. Hauptgruppe, mindestens ein Element der III. und/oder IV. Hauptgruppe und mindestens ein Element der III. Nebengruppe einschließlich der Lanthaniden und Actiniden.

Beispielsweise können erfindungsgemäß alle Dehydrierkatalysatoren eingesetzt werden, die in den WO 99/46039, US 4,788,371, EP-A 705 136, WO 99/29420, US 5,220,091, US 5,430,220, US 5,877,369, EP 0 117 146, DE-A 199 37 106, DE-A 199 37 105 und DE-A 199 37 107 offenbart werden. Besonders bevorzugte Katalysatoren für die vorstehend beschriebenen Varianten der autothermen Propan-Dehydrierung sind die Katalysatoren gemäß den Beispielen 1, 2, 3 und 4 der DE-A 199 37 107.

Die autotherme Alkan-Dehydrierung wird bevorzugt in Gegenwart von Wasserdampf durchgeführt. Der zugesetzte Wasserdampf dient als Wärmeträger und unterstützt die Vergasung von organischen Ablagerungen auf den Katalysatoren, wodurch der Verkokung der Katalysatoren entgegengewirkt und die Standzeit der Katalysatoren erhöht wird. Dabei werden die organischen Ablagerungen in Kohlenmonoxid, Kohlendioxid und gegebenenfalls Wasser umgewandelt.

Der Dehydrierungskatalysator kann in an sich bekannter Weise regeneriert werden. So kann dem Reaktionsgasgemisch Wasserdampf zugesetzt werden oder von Zeit zu Zeit ein Sauerstoff enthaltendes Gas bei erhöhter Temperatur über die Katalysatorschüttung geleitet werden und der abgeschiedene Kohlenstoff abgebrannt werden. Durch die Verdünnung mit Wasserdampf wird das Gleichgewicht zu den Produkten der Dehydrierung verschoben. Gegebenenfalls wird der Katalysator nach der Regenerierung mit einem wasserstoffhaltigen Gas reduziert.

Bei der autothermen Propan-Dehydrierung unter Einspeisung von im Wesentlichen reinem Sauerstoff wird ein Gasgemisch erhalten, welches im Allgemeinen folgende Zusammensetzung aufweist: 10 bis 45 Vol.-% Propan, 5 bis 40 Vol.-% Propen, 0 bis 5 Vol.-% Methan, Ethan, Ethen und C₄⁺-Kohlenwasserstoffe, 0 bis 5 Vol.-% Kohlendioxid, 0 bis 20 Vol.-% Wasserdampf und 0 bis 25 Vol.-% Wasserstoff sowie 0 bis 5 Vol.-% Inertgase.

Bei der autothermen Butan-Dehydrierung unter Einspeisung von im Wesentlichen reinem Sauerstoff wird ein Gasgemisch erhalten, welches im Allgemeinen folgende Zusammensetzung aufweist: 5 bis 40 Vol.-% Butan, 10 bis 60 Vol.-% 1-Buten und 2-Buten, 0 bis 10 Vol.-% Methan, Ethan, Ethen, Propan, Propen und C₅⁺-Kohlenwasserstoffe, 0 bis 5 Vol.-% Kohlendioxid, 0 bis 20 Vol.-% Wasserdampf und 0 bis 25 Vol.-% Wasserstoff sowie 0 bis 5 Vol.-% Inertgase.

Der Produktgasstrom b steht beim Verlassen der Dehydrierzone im Allgemeinen unter einem Druck von 1 bis 5 bar, vorzugsweise 1,5 bis 3 bar, und weist eine Temperatur im Bereich von 400 bis 700°C auf.

Der Produktgasstrom b kann in zwei Teilströme aufgetrennt werden, wobei ein Teilstrom in die autotherme Dehydrierung zurückgeführt wird, entsprechend der in DE-A 102 11 275 und DE-A 100 28 582 beschriebenen Kreisgasfahrweise.

Im Verfahrensteil C) wird der Produktgasstrom b komprimiert. Die Komprimierung des Produktgasstroms b erfolgt auf Drücke von 5 - 150 bar, bevorzugt auf 15 - 100 bar, besonders bevorzugt auf 20 - 60 bar. Die Verdichtung kann mehrstufig mit Zwischenkühlungen erfolgen, beispielsweise in drei oder vier Stufen, vorzugsweise erfolgt sie mehrstufig, beispielsweise dreistufig. In einer Ausführungsform des erfindungsgemäßen Verfahrens erfolgt eine ein- oder zweistufige Kompression des Produktgasstroms b auf einen Druck im Bereich von 5 bis 12 bar und anschließend eine ein- oder zweistufige Kompression auf einen Druck im Bereich von 10 bis 25 bar. Auch die Kühlung kann mehrstufig erfolgen, bevorzugt erfolgt sie mehrstufig. Als Kühlmittel kommen Luft in Luftkühlern, Fluss- oder Kaltwasser sowie Kältemittel wie Ethen, Propen und Propan, welche durch Verdichten auf Drücke bis zu 20 bar und anschließendes Entspannen auf Temperaturen im Bereich von -40°C bis -100°C abgekühlt werden, zum Einsatz.

Optional wird der Produktgasstrom b in eine wässrige Phase c1, eine Kohlenwasserstoffphase c2 enthaltend das Alken (III) sowie das nicht umgesetzte Alkan (II) und eine Gasphase c3 enthaltend Wasserstoff und Leichtsieder aufgetrennt.

Der Abtrennschritt innerhalb des Verfahrensteils C) erfolgt im Allgemeinen, wenn der Produktgasstrom b Wasserdampf enthält. Es kann aber auch lediglich eine Wasserabtrennung erfolgen (siehe unten).

Aus dem Produktgasstrom b kann zunächst Wasser abgetrennt werden. Die Abtrennung von Wasser kann durch Kondensieren lassen durch Abkühlen und gegebenenfalls Verdichten des Produktgasstroms b durchgeführt werden und kann in einer oder mehreren Abkühlungs- und gegebenenfalls Verdichtungsstufen durchgeführt werden. Im Allgemeinen wird der Produktgasstrom b hierzu auf eine Temperatur im Bereich von 30 bis 80°C, vorzugsweise 40 bis 65°C abgekühlt. Das Kondensieren lassen kann dabei vor der Kompression und/oder in den Verdichtungsstufen als Zwischenkühlung erfolgen.

In einer Ausführungsform des erfindungsgemäßen Verfahrens wird der Produktgasstrom b durch eine Kaskade von Wärmetauschern geleitet und so zunächst auf eine Temperatur im Bereich von 50 bis 200°C abgekühlt und anschließend in einem Quenchturm mit Wasser auf eine Temperatur von 40 bis 80°C, beispielsweise 55°C, weiter abgekühlt. Dabei kondensiert der größte Teil des Wasserdampfs aus, aber auch ein Teil der im Produktgasstrom b enthaltenen Hochsieder. Im Falle der Propan-Dehydrierung können dies C₄⁺-Kohlenwasserstoffe, insbesondere die C₅⁺-Kohlenwasserstoffe sein.

Dabei wird ein an Wasserdampf abgereicherter Produktgasstrom b erhalten. Dieser enthält im Allgemeinen noch bis zu 5 Vol.-% Wasserdampf. Zur praktisch vollständigen Entfernung von Wasser aus dem Produktgasstrom b kann eine Trocknung mittels Molekularsieb vorgesehen werden.

Wird die autotherme Alkan-Dehydrierung unter Einspeisung von reinem Sauerstoff oder mit Sauerstoff angereicherter Luft als sauerstoffhaltigem Gas durchgeführt, so kann die Aufarbeitung des Produktgasstroms b und die Gewinnung des Alkan und Alken enthaltenden Gemischs c2 auch wie nachstehend beschrieben erfolgen.

Anschließend wird der Produktgasstrom b abgekühlt und ein flüssiger Kohlenwasserstoffstrom c2 enthaltend Propan und Propen beziehungsweise n-Butan und Butene durch Kondensieren lassen abgetrennt, wobei ein Restgasstrom c3 enthaltend Wasserstoff und Leichtsieder verbleibt. Der Kohlenwasserstoffstrom c2 kann im Falle der Propan-Dehydrierung daneben Methan, Ethan, Ethen und C₄⁺-Kohlenwasserstoffe enthalten, im Allgemeinen enthält er zumindest in geringen Mengen Ethan und Ethen. Die Temperatur und der Druck im Abtrennschritt innerhalb der Kompressionsstufe C können auch so gewählt werden, dass ein Großteil der im Produktgasstrom b enthaltenen Alkane und Alkene im Gastrom c3 enthalten sind. Dieser Gastrom c3 kann entweder ebenso wie der Kohlenwasserstoffstrom c2 in die Esterbildungszone D oder alternativ in eine Absorptionstufe (wie unten beschrieben) geführt werden.

Im Falle der n-Butan-Dehydrierung werden die Bedingungen so gewählt, dass ganz überwiegend n-Butan und Butene auskondensieren. Der Restgasstrom c3 enthält neben Wasserstoff als Leichtsieder im Allgemeinen noch Methan und Kohlenmonoxid. Daneben kann er auch noch Ethan und Ethen und - falls die autotherme Dehydrierung nicht unter Einspeisung von reinem Sauerstoff durchgeführt wird - insbesondere Stickstoff und Edelgase (hauptsächlich Argon) enthalten. Daneben kann er auch noch C₃- und C₄-Kohlenwasserstoffe enthalten. Dazu wird der Produktgasstrom b im Allgemeinen auf einen Druck im Bereich von 5 bis 60 bar verdichtet und auf eine Temperatur im Bereich von -10 bis -60°C abgekühlt.

Neben den Kohlenwasserstoffstrom c2 kann beim Abkühlen und Verdichten eine wässrige Phase c1 auskondensieren und durch Phasentrennung in einem Phasenscheider von der C₃-Kohlenwasserstoffphase c2 abgetrennt werden, falls keine vollständige Wasserabtrennung aus dem Produktgastrom b vor dem Kondensationsschritt erfolgt ist. Bei mehrstufigem Abkühlen und Verdichten können sämtliche anfallenden Kondensatströme dem Phasenscheider zugeführt werden.

Auf eine vorherige Entfernung von Wasser aus dem Produktgasstrom b vor dem Kondensieren lassen der C₃-Kohlenwasserstoffphase c2 kann auch verzichtet werden. Dann kondensiert Wasser als wässrige Phase c1 zusammen mit der Alkan und Alken enthaltenden Kohlenwasserstoffphase c2 aus. Wässrige Phase und Kohlenwasserstoffphase werden dann anschließend in einem Phasenscheider getrennt.

Im Allgemeinen erfolgt das Abkühlen des Produktgasstroms durch Wärmetausch mit einem Kühlmittel. Die Abkühlung kann mehrstufig unter Einsatz mehrerer Kühlkreisläufe erfolgen. Die Abkühlung kann mehrstufig in einer Kolonne erfolgen, wobei das in der Kolonne aufsteigende Gas entnommen, abgekühlt, (teilweise) kondensiert und in die Kolonne zurückgeführt wird. Am Kolonnensumpf wird das Kondensat, am Kolonnenkopf das nicht kondensierte Gas entnommen, welches auch im obersten Kühlkreislauf nicht kondensiert ist.

Wird die Alkan-Dehydrierung als autotherme Dehydrierung unter simultaner Verbrennung des gebildeten Wasserstoffs durchgeführt, so resultiert ein geringer Wasserstoffgehalt des Produktgasstroms b. Als Folge davon können in dem Abtrennschritt C) - falls dieser durchgeführt wird - die C₃- beziehungsweise C₄-Kohlenwasserstoffe ganz überwiegend auskondensiert werden und es wird nur ein sehr kleiner Teil der C₃- beziehungsweise C₄-Kohlenwasserstoffe mit dem Wasserstoff/Leichtsieder enthaltenden Abgasstrom c3 ausgetragen.

Vor Durchführung der Kohlenwasserstoff-Kondensation kann aus dem Produktgasstrom b Kohlendioxid durch Gaswäsche abgetrennt werden, wobei ein an Kohlendioxid abgereicherter Produktgasstrom b erhalten wird. Der Kohlendioxid-Gaswäsche kann eine gesonderte Verbrennungsstufe vorgeschaltet werden, in der Kohlenmonoxid selektiv zu Kohlendioxid oxidiert wird.

Zur CO₂-Abtrennung wird im Allgemeinen Natronlauge, Kalilauge oder eine Alkanolamin-Lösung als Waschflüssigkeit eingesetzt, bevorzugt wird eine aktivierte N-Methyldiethanolamin-Lösung eingesetzt. Im Allgemeinen wird vor Durchführung der Gaswäsche der Produktgasstrom c durch ein- oder mehrstufige Verdichtung auf einen Druck im Bereich von 5 bis 25 bar verdichtet.

Es kann ein an Kohlendioxid abgereicherter Produktgasstrom b mit einem CO₂-Gehalt von im Allgemeinen < 100 ppm oder sogar < 10 ppm erhalten werden.

Der in dem Kühl- und Kondensationsschritt C) erhaltene flüssige Kohlenwasserstoff-Kondensatstrom c2 enthält im Allgemeinen 20 bis 60 mol-% Alkan (II), 20 bis 60 mol-% Alken (III), 0 bis 20 mol-% Leichtsieder und 0 bis 5 mol-% Hochsieder.

Im Falle der Propan-Dehydrierung kann der im Kühl- und Kondensationsschritt C) erhaltene flüssige Kohlenwasserstoff-Kondensatstrom c2 20 bis 70 mol-% Propan, 20 bis 60 mol-% Propen, 0 bis 10 mol-% Methan, 0 bis 10 mol-% Ethan und Ethen und 0 bis 5 mol-% C₄⁺-Kohlenwasserstoffen enthalten.

Wird die autotherme Alkan-Dehydrierung unter Einspeisung von Luft als sauerstoffhaltigem Gas durchgeführt, so kann die Aufarbeitung des Produktgasstroms b und die Gewinnung des Alkan und Alken enthaltenden Gemischs c2 auch wie nachstehend beschrieben erfolgen. Zunächst wird Wasserdampf durch Kondensieren lassen abgetrennt, indem der Produktgasstrom b abgekühlt und gegebenenfalls verdichtet wird, wobei ein an Wasserdampf abgereicherter Produktgasstrom b erhalten wird. Anschließend werden Alkan und Alken von nicht kondensierbaren oder leicht siedenden Gasbestandteilen durch Inkontaktbringen des Produktgasstroms b mit einem inerten Absorptionsmittel und anschließender Desorption des in dem inerten Absorptionsmittel gelösten Alkans und Alkens abgetrennt, wobei ein gasförmiger C₃-beziehungsweise C₄-Kohlenwasserstoffstrom erhalten und der Abgasstrom c3, enthaltend Wasserstoff und Leichtsieder (im Falle von der Propan-Dehydrierung Methan, Ethan, Ethen, Stickstoff, Kohlenmonoxid, Kohlendioxid, gegebenenfalls Sauerstoff und gegebenenfalls Inertgase, im Falle der n-Butan-Dehydrierung zusätzlich auch Propan und Propen), abgetrennt wird. Die beschriebene Aufarbeitung des Produktgasstroms b kann auch im Falle der autothermen Alkan-Dehydrierung unter Einspeisung von reinem Sauerstoff beziehungsweise mit Sauerstoff angereicherter Luft als sauerstoffhaltigem Gas entsprechend durchgeführt werden.

Dazu wird in der Absorptionsstufe bei 5 - 40 bar, bevorzugt 8 - 20 bar, besonders bevorzugt 10 - 15 bar der Gasstrom b mit einem inerten Absorptionsmittel in Kontakt gebracht, wobei die C₃- beziehungsweise C₄-Kohlenwasserstoffe und auch geringe Mengen der C₂-Kohlenwasserstoffe in dem inerten Absorptionsmittel absorbiert werden und ein mit C₃- beziehungsweise C₄-Kohlenwasserstoffen beladenes Absorptionsmittel und ein die übrigen Gasbestandteile enthaltendes Abgas c3 erhalten werden. Im Wesentlichen sind dies Kohlenstoffoxide, Wasserstoff, Inertgase sowie C₂-Kohlenwasserstoffe und Methan. Auch gewisse Mengen Propan und Propen beziehungsweise C₄-Kohlenwasserstoffe können noch in dem Strom c3 enthalten sein, da die Abtrennung im Allgemeinen nicht ganz vollständig ist. In einer Desorptionsstufe werden die C₃-beziehungsweise C₄-Kohlenwasserstoffe aus dem Absorptionsmittel wieder freigesetzt.

In der Absorptionsstufe eingesetzte inerte Absorptionsmittel sind im Allgemeinen hochsiedende unpolare Lösungsmittel, in denen das abzutrennende C₃- beziehungsweise C₄-Kohlenwasserstoff-Gemisch eine deutlich höhere Löslichkeit als die übrigen abzutrennenden Gasbestandteile aufweist. Die Absorption kann durch einfaches Durchleiten des Stroms c durch das Absorptionsmittel erfolgen. Sie kann aber auch in Kolonnen erfolgen. Dabei kann im Gleichstrom, Gegenstrom oder Kreuzstrom gearbeitet werden. Geeignete Absorptionskolonnen sind z.B. Bodenkolonnen mit Glocken-, Ventil- und/oder Siebböden, Kolonnen mit strukturierten Packungen, z.B. Gewebepackungen oder Blechpackungen mit einer spezifischen Oberfläche von 100 bis 1000 m²/m³ wie Mellapak^{®} 250 Y, und Füllkörperkolonnen, z.B. mit Kugeln, Ringen oder Sätteln aus Metall, Kunststoff oder Keramik als Füllkörpern. Es kommen aber auch Riesel- und Sprühtürme, Graphitblockabsorber, Oberflächenabsorber wie Dickschicht- und Dünnschichtabsorber sowie Blasensäulen mit und ohne Einbauten in Betracht.

Vorzugsweise weist die Absorptionskolonne einen Absorptionsteil und einen Rektifikationsteil auf. Zur Erhöhung der Anreicherung der C₃- beziehungsweise C₄-Kohlenwasserstoffen im Lösungsmittel nach Art einer Rektifikation kann dann in den Kolonnensumpf Wärme eingetragen werden. Alternativ kann in den Kolonnensumpf ein Strippgasstrom eingespeist werden, beispielsweise aus Stickstoff, Luft, Wasserdampf oder Propan/Propen-Gemischen. So wird im Rektifikationsteil der Absorptionskolonne das beladene Absorptionsmittel mit dem Strippgasstrom in Kontakt gebracht. Dadurch werden aus dem beladenen Absorptionsmittel C₂⁻-Kohlenwasserstoffe herausgestrippt. Ein Teil des Kopfproduktes kann kondensiert und als Rücklauf wieder auf den Kolonnenkopf gegeben werden, um Lösungsmittelverluste zu begrenzen.

Geeignete Absorptionsmittel sind vergleichsweise unpolare organische Lösungsmittel, beispielsweise aliphatische C₄-C₁₈-Alkene, Naphtha oder aromatische Kohlenwasserstoffe wie die Mittelölfraktionen aus der Paraffindestillation, oder Ether mit sperrigen Gruppen, oder Gemische dieser Lösungsmittel, wobei diesen ein polares Lösungsmittel wie 1,2-Dimethylphthalat zugesetzt sein kann. Geeignete Absorptionsmittel sind weiterhin Ester der Benzoesäure und Phthalsäure mit geradkettigen C₁-C₈-Alkanolen, wie Benzoesäure-n-butylester, Benzoesäuremethylester, Benzoesäureethylester, Phthalsäuredimethylester, Phthalsäurediethylester, sowie so genannte Wärmeträgeröle, wie Biphenyl und Diphenylether, deren Chlorderivate sowie Triarylalkene. Ein geeignetes Absorptionsmittel ist ein Gemisch aus Biphenyl und Diphenylether, bevorzugt in der azeotropen Zusammensetzung, beispielsweise das im Handel erhältliche Diphyl^{®}. Häufig enthält dieses Lösungsmittelgemisch Dimethylphthalat in einer Menge von 0,1 bis 25 Gew.-%. Geeignete Absorptionsmittel sind ferner Butane, Pentane, Hexane, Heptane, Octane, Nonane, Decane, Undecane, Dodecane, Tridecane, Tetradecane, Pentadecane, Hexadecane, Heptadecane und Octadecane oder aus Raffinerieströmen gewonnene Fraktionen, die als Hauptkomponenten die genannten linearen Alkane enthalten. Bevorzugte Absorptionsmittel sind C₈-C₁₀-Kohlenwasserstoffe, besonders bevorzugt sind C₉-Kohlenwasserstoffe, insbesondere Nonane.

Zur Desorption der C₃- beziehungsweise C₄-Kohlenwasserstoffe wird das beladene Absorptionsmittel erhitzt und/oder auf einen niedrigeren Druck entspannt. Alternativ dazu kann die Desorption auch durch Strippung, üblicherweise mit Wasserdampf, oder in einer Kombination von Entspannung, Erhitzen und Strippung in einem oder mehreren Verfahrensschritten erfolgen. Beispielsweise kann die Desorption zweistufig durchgeführt werden, wobei die zweite Desorptionsstufe bei einem niedrigeren Druck als die erste Desorptionsstufe durchgeführt wird und das Desorptionsgas der zweiten Stufe in die Absorptionsstufe zurückgeführt wird. Das in der Desorptionsstufe regenerierte Absorptionsmittel wird in die Absorptionsstufe zurückgeführt. Gegebenenfalls wird ein Teil dieses Absorptionsmittelstroms, der C₄⁺-Kohlenwasserstoffe enthalten kann, ausgeschleust, aufgearbeitet und zurückgeführt, oder verworfen.

In einer Verfahrensvariante wird der Desorptionsschritt durch Entspannung und/oder Erhitzen des beladenen Absorptionsmittels durchgeführt. In einer weiteren Verfahrensvariante wird zusätzlich mit Wasserdampf gestrippt.

Die Abtrennung ist im Allgemeinen nicht ganz vollständig, so dass in dem C₃-beziehungsweise C₄- Kohlenwasserstoffstrom - je nach Art der Abtrennung - noch geringe Mengen oder auch nur Spuren der weiteren Gasbestandteile, insbesondere der leicht siedenden Kohlenwasserstoffe, vorliegen können.

Anschließend kann der desorbierte C₃- beziehungsweise C₄-Kohlenwasserstoffstrom abgekühlt werden, wobei er zusätzlich in einer oder mehreren weiteren Kompressionsstufen komprimiert werden kann. Dabei wird der flüssige Kondensatstrom c2 aus C₃-beziehungsweise C₄-Kohlenwasserstoffen erhalten. Der Strom c2 kann noch geringe Mengen C₂-Kohlenwasserstoffe enthalten. Daneben können ein wässriger Kondensatstrom und gegebenenfalls weitere Mengen des Abgasstroms c3 anfallen. Der wässrige Kondensatstrom fällt im Allgemeinen an, wenn zur Desorption der gelösten Gase mit Wasserdampf gestrippt wird.

Die Kompression kann wiederum ein- oder mehrstufig erfolgen. Im Allgemeinen wird insgesamt von einem Druck im Bereich von 1 bis 29 bar, vorzugsweise 1 bis 10 bar, auf einen Druck im Bereich von 12 bis 30 bar komprimiert. Nach jeder Kompressionsstufe folgt eine Abkühlstufe, in der der Gasstrom auf eine Temperatur im Bereich von 15 bis 80°C, vorzugsweise 15 bis 60°C, abgekühlt wird. Anschließend wird das komprimierte Gasgemisch auf eine Temperatur von -10°C bis 60°C, vorzugsweise -10°C bis 30°C abgekühlt. Ein gegebenenfalls vorhandener wässriger Kondensatstrom kann in einem Phasentrennapparat von dem flüssigen C₃- beziehungsweise C₄-Kohlenwasserstoffstrom abgetrennt werden.

Die oben beschriebene Absorptionsstufe kann auch wie folgt ausgeführt werden:

Als Absorptionsmittel wird in der Absorptionskolonne die gleiche organische Säure eingesetzt, die in der Esterbildungszone mit dem entsprechenden Alken umgesetzt wird. Dabei kann dann auf den oben beschriebenen Desorptionsschritt verzichtet werden. Das mit C₃- beziehungsweise C₄-Kohlenwasserstoffen beladene Absorptionsmittel, d.h. in diesem Fall die organische Säure (z.B. Essigsäure oder Ameisensäure), kann dann, gegebenenfalls nach weiterem Aufheizen und/oder Komprimieren, direkt in die Esterbildungszone geführt werden. Als Absorptionsmittelzulauf in die Absorptionskolonne kann in diesem Fall die aus dem Abtrennschritt (G) nach der Esterspaltungszone abgetrennte organische Säure verwendet werden. Diese wird dann nicht direkt in die Esterbildungszone, sondern in die Absorptionsstufe und von dort, beladen mit den C₃- beziehungsweise C₄-Kohlenwasserstoffen, in die Esterbildungszone zurückgeführt.

Der Abtrennschritt innerhalb des Verfahrensteils C) kann, muss aber nicht durchgeführt werden. Wie oben beschrieben, wird aber immer zumindest eine Kompression des Produktgasstroms b durchgeführt. Zum Beispiel kann, falls die Alkan-Dehydrierung nicht unter Einspeisung eines sauerstoffhaltigen Gases und auch nicht in Gegenwart von Wasserdampf durchgeführt wird, das erhaltene Dehydriergasgemisch b, das im Falle der Propan-Dehydrierung im Wesentlichen aus Propan, Propen, Wasserstoff und Leichtsiedern und im Falle der n-Butan-Dehydrierung im Wesentlichen aus Butan, 1-Buten, 2-Buten, Wasserstoff und Leichtsiedern besteht, direkt und ohne vorherige Abtrennung der C₃- beziehungsweise C₄-Kohlenwasserstoffe in die Esterbildungszone eingespeist und mit der organischen Säure in Kontakt gebracht werden. Enthält der Produktgasstrom b Wasserdampf, da die Dehydrierung unter Einspeisung von Sauerstoff und/oder unter Einspeisung von Wasserdampf durchgeführt wurde, kann eine Abtrennung von Wasserdampf alleine - beispielsweise durch Kondensation wie oben unter C) beschrieben - ausreichend sein und das verbliebene, die C₃- beziehungsweise C₄-Kohlenwasserstoffe, Wasserstoff und Leichtsieder enthaltende Gemisch gasförmig oder flüssig mit der organischen Säure umgesetzt werden. Auch die Gegenwart von Kohlenstoffoxiden und weiteren Inertgasen (Luftstickstoff) stören die Esterbildungsreaktion grundsätzlich nicht.

Der Restgasstrom c3 kann, bevorzugt wird er überwiegend in die Dehydrierstufe A) zurückgeführt. Ein Teilstrom wird abgetrennt und aus dem Prozess ausgeschleust, um eine Anreicherung von Nebenkomponenten zu vermeiden. Dieser Teilstrom kann verbrannt werden oder einer Verfahrensstufe zur Rückgewinnung von in diesem enthaltenen Alkan/Alken zugeführt werden. Die Rückgewinnung kann als Ab- oder Adsorption, als Membrantrennung oder Rektifikation durchgeführt werden.

Ein Teilstrom des Restgasstroms c3 kann auch dem Esterbildungsschritt D) zugeführt werden.

Die Kohlenwasserstoffphase c2 kann direkt oder nach weiterer Druckerhöhung der Esterbildungsstufe D) zugeführt werden. Der wässrige Kondensatstrom c1 kann aus dem Verfahren ausgeschleust oder in die Esterspaltungs-Stufe (Verfahrensteil F)) geführt werden.

In einer Verfahrensstufe D) wird der Produktgasstrom b beziehungsweise - falls der Auftrennschritt in der Verfahrensstufe C) durchgeführt wird - die das Alkan (II) und das Alken (III) enthaltende Phase c2 und gegebenenfalls der Restgasstrom c3 oder, falls der Absorptionsschritt wie oben beschrieben mit der organischen Säure durchgeführt wird, die mit C₃- beziehungsweise C₄-Kohlenwasserstoffen beladene organische Säure in einer Esterbildungszone mit einer organischen Säure (IV) umgesetzt, wobei ein Produktgemisch d enthaltend den entsprechenden Alkylester (V) (Isopropylester beziehungsweise 2-Butylester) der organischen Säure (IV) und nicht umgesetztes Alkan (II) erhalten wird.

Die Esterbildung kann in flüssiger Phase oder zweiphasig (bezüglich der Reaktanten) als Gas/Flüssig-Reaktion durchgeführt werden.

Die Esterbildung wird im Allgemeinen bei einem Druck von 10 bis 100 bar und bei einer Temperatur von 50 bis 250°C durchgeführt. Im Allgemeinen wird die organische Säure, bezogen auf Alken, in Mengen von 0,5 - 50 mol pro mol Alken, bevorzugt in stöchiometrischem Überschuss, bevorzugt in Mengen von 1,1 - 6 , besonders bevorzugt 1,2 - 2,5 mol pro mol Alken, eingesetzt. Im Allgemeinen reagieren 50 bis 90 % des Alkens zum entsprechenden Alkylester. Daneben können in dem Produktgasstrom b beziehungsweise in dem Strom c2 enthaltene ungesättigte Nebenkomponenten zu Alkylestern reagieren. Diese können in den sich anschließenden Aufarbeitungsschritten ohne weiteres destillativ abgetrennt werden. Die im Strom b beziehungsweise c2 enthaltenen Alkane reagieren naturgemäß in der Esterbildungstufe D) nicht und verlassen die Esterbildungsstufe D) gemeinsam mit in der Esterbildung nicht umgesetztem Alken im Fall des Betriebs der Esterbildungstufe D) in homogener flüssiger Phase im Strom d oder im Fall des Betriebs der Esterbildungstufe D) in Gas-Flüssig-Fahrweise als zusätzlicher Strom d2). Dieser Strom kann neben Alkan und Alken noch geringe Mengen des gebildeten Esters sowie Wasser sowie die bereits in Strom c2 enthaltenen Leichtsieder enthalten.

Wird die Esterbildungsstufe D) in Gas-Flüssig-Fahrweise betrieben, kann sie zwei Zonen, eine Reaktionszone und eine Rückwaschzone, umfassen. Ziel der Reaktionszone ist ein möglichst hoher Umsatz der Säure mit dem Alken. In der Rückwaschzone wird durch Zugabe von Wasser der Gasstrom d2 weitgehend von Säureresten befreit. Dadurch können Korrosionsprobleme in anderen Anlagenteilen bei der Rückführung des Stroms d2) vermieden werden.

Die Esterbildung kann in Festbettreaktoren in Rieselfahrweise oder in WirbelschichtFahrweise durchgeführt werden. Prinzipiell können die organische Säure und der alkenhaltige Strom im Gleich- oder Gegenstrom, aber auch im Kreuzstrom, geführt werden. Bevorzugt erfolgt eine Kaskadierung von mehreren Katalysatorbetten, gegebenenfalls mit Zwischeneinspeisung der Säure und/oder des alkenhaltigen Stromes. Bevorzugt wird eine Kaskade von 2-5, besonders bevorzugt 2-3 verschiedenen Reaktionszonen verwendet. Die freiwerdende Reaktionswärme kann durch innenliegende Wärmetauscherflächen abgeführt werden. Auch eine Fahrweise mit einem externen Umwälzkreis, in dem ein Wärmetauscher zur Abfuhr der Reaktionswärme angebracht ist, ist möglich. Durch die Einstellung der Umwälzmenge kann das axiale Temperaturprofil im Reaktor nahezu beliebig eingestellt werden. Alternativ kann die Esterbildung auch in Blasensäulen oder Strahlschlaufenreaktoren durchgeführt werden. Eine besondere Ausführungsform ist der Rohrreaktor in Wirbelschichtfahrweise, der gezielt am Lockerungspunkt des Katalysators betrieben wird (so genannte Schwebebettfahrweise), mit externem Wärmetauscher und 2-facher Kaskadierung.

In der Rückwaschzone erfolgt durch Zugabe von Wasser eine Entfernung von Säureresten. Apparativ entspricht diese Zone einer Absorptionskolonne, die separat als eigener Apparat oder integriert in den Festbettreaktor ausgeführt sein kann. Als Einbauten können Böden, Packungen oder regellose Schüttungen verwendet werden. Die Menge des Waschwassers wird dabei so gewählt, dass die Säurerestmenge im Gastrom d2 < 50 ppm, bevorzugt « 10 ppm besonders bevorzugt « 1 ppm beträgt. Ein typisches Verhältnis Waschwassermenge : zu entfernende Säuremenge zur Erreichung von < 1 ppm Säure liegt im Bereich von 1 kg/kg bis 20 kg/kg. Das mit Säureresten beladene Waschwasser kann in der Verfahrensstufe F) zur Esterspaltung eingesetzt werden.

Geeignete heterogene Esterbildungskatalysatoren sind saure lonentauscherharze, insbesondere solche aus sulfoniertem, mit Divinylbenzol vernetztem Polystyrol. Diese Katalysatoren können unterschiedliche Porenstrukturen aufweisen - man unterscheidet mikroporöse, gelartige und makropöröse Katalysatoren. Außerdem können an den aromatischen Ringen des Polystyrols elektronegative Reste, z.B. Chlor oder Fluor, gebunden sein. Geeignete Katalysatoren dieser Art sind beispielsweise Amberlyst 15, 16, 36, 39, 40, 46, 48, 70, 119, 139 Lewatit K1131, K1221, K1461, K2420, K2629, K2649, Purolite CT169, CT175, CT275, Diaion RCP145H. Besonders bevorzugt sind solche Katalysatoren, die einen hohen Gehalt saurer Gruppen enthalten wie z. B. Amberlyst 35, 36, 40, 49, 119, Lewatit K2649, Purolite CT275. Die hier genannten Katalysatoren sind üblicherweise in trockener oder in wasserhaltiger Form erhältlich. Beide Formen sind geeignet, bei den wasserhaltigen Katalysatoren wird das Wasser durch Waschen mit der organischen Säure verdrängt. Eine den sauren lonenaustauscherharzen verwandte Gruppe von Katalysatoren leitet sich von sulfonierten polykondensierten Aromaten beziehungsweise graphitischem Kohlenstoff ab. Solche Materialien werden z. B. durch Sulfonierung polyzyklischer Aromaten, wie z.B. Naphthalin oder Anthracen, unter Bedingungen, die zu einer Kondensation der Aromaten führen, gebildet. Ein ähnliches Verfahren geht von der Verkohlung organischen Materials, z.B. von Zuckern, unter anaeroben Bedingungen aus. Die entsprechenden Rückstände werden anschließend sulfoniert. Eine weitere Gruppe organischer heterogener Katalysatoren leitet sich von ionischen Flüssigkeiten ab, die auf geeigneten Trägermaterialien adsorbiert werden. Neben den polymeren lonenaustauscherharzen eignen sich weiterhin auch eine Reihe anorganischer Katalysatoren wie saure Metalloxidkatalysatoren oder saure Zeolithe. Zu den sauren Metalloxidkatalysatoren sind insbesondere sulfatiertes

Zirkoniumoxid und Zirkoniumwolframat- beziehungsweise Titanwolframat-Systeme zu rechnen. Weiterhin gehören zu dieser Gruppe von Katalysatoren die in Wasser unlöslichen sauren Salze der Heteropolysäuren, z.B. der Wolframato- oder Molybdatophosphorsäure oder der Wolframatokieselsäure. Solche unlöslichen Salze bilden sich aus diesen Säuren mit Metallkationen mit großen Ionenradien, wie z.B. K⁺, Rb⁺, Cs⁺, NH₄⁺ oder Ag⁺. In diesen Salzen werden üblicherweise 10 - 90 mol-%, insbesondere 40 - 85 mol-%, der sauren Zentren gegen Kationen ausgetauscht. Eine weitere Gruppe von Katalysatoren leitet sich von Heteropolysäuren oder deren Salzen ab, die auf einem inerten Trägermaterial wie z.B. Kieselgel, Aluminiumoxid oder Aktivkohle adsorbiert sind. Zu den geeigneten Zeolith-Katalysatoren gehören solche des Strukturtyps der Beta-Zeolithe, der Faujasite, der Mordenite und der ZSM-5-Zeolithe. Neben der Zeolithstruktur ist das Verhältnis der Si/AI-Atome (der Modul) im Zeolithgerüst für die katalytische Aktivität des Zeolithen auschlaggebend. Für das erfindungsgemäße Verfahren eignen sich solche Zeolithe, die einen Modul zwischen 2 und 500 besitzen, insbesondere zwischen 3 und 200 und ganz besonders bevorzugt zwischen 5 und 100. Die hier beschriebenen anorganischen Katalysatoren werden üblicherweise thermisch aktiviert, d.h. die Materialien werden bei Temperaturen zwischen 50 und 900°C, vorzugsweise zwischen 90 und 500°C kalziniert.

Die Esterbildungsreaktion kann auch homogen katalysiert erfolgen. Hierfür eignen sich Mineralsäuren, insbesondere Schwefelsäure, Sulfonsäuren oder die freien Heteropolysäuren sowie deren saure lösliche Salze oder saure ionische Flüssigkeiten.

Bevorzugt erfolgt die Esterbildung in Gegenwart heterogener Katalysatoren. Bevorzugte heterogene Katalysatoren sind saure lonenaustauscherharze, die sauren K, Cs, oder Ammonium-Salze der Heteropolysäuren sowie Beta-Zeolithe und Faujasite. Besonders bevorzugt sind lonentauscherharze.

Bevorzugte organische Säuren (IV) sind Ameisensäure und Essigsäure, welche mit Propen zu Isopropylformiat beziehungsweise Isopropylacetat und mit Butenen (1-Buten und 2-Buten) zu 2-Butylformiat beziehungsweise 2-Butylacetat reagieren.

In der Verfahrensstufe E) wird von dem Produktgemisch d der Esterbildung ein das nicht umgesetzte Alkan (II) (Propan beziehungsweise n-Butan) enthaltender Gasstrom e1 abgetrennt, der gegebenenfalls in die Dehydrierzone zurückgeführt wird. Hierzu wird im Allgemeinen das Produktgemisch d der Esterbildung entspannt, wobei ein Propan beziehungsweise n-Butan enthaltender Gasstrom e1 abgetrennt und ein den Alkylester (V) enthaltendes Produktgemisch e2 erhalten wird. Der Gasstrom e1 kann mit dem Gasstrom d2 der Rückwaschzone im Verfahrensteil D vereint und gemeinsam der Dehydrierzone B zugeführt werden.

Im Allgemeinen wird das Produktgemisch d von einem Druck im Bereich von 20 bis 60 bar auf einen Druck im Bereich von 2 bis 10 bar entspannt. Mit dem Propan beziehungsweise n-Butan werden dabei gegebenenfalls in dem Produktgemisch d enthaltene Leichtsieder wie Ethan, Ethen, Methan, Kohlenstoffoxide und Inertgase mit abgetrennt. Der das Alkan (II) enthaltende Gasstrom e1 wird vorzugsweise in die Alkan-Dehydrierung zurückgeführt.

Der das Alkan (II) enthaltende Gasstrom e1 kann noch Leichtsieder wie Ethan, Ethen, Methan, Kohlenstoffoxide und Inertgase sowie Wasserstoff enthalten. Im Allgemeinen enthält er Leichtsieder und gegebenenfalls Wasserstoff, wenn nicht bereits in Schritt C) die vorstehend beschriebene (optionale) Abtrennung von Leichtsiedern und Wasserstoff (Restgasstrom c3) erfolgt ist. Von dem Gasstrom e1 kann ein Teilstrom abgetrennt und aus dem Prozess ausgeschleust werden, um eine Anreicherung von Nebenkomponenten zu vermeiden. Dieser Teilstrom kann verbrannt werden oder einer Verfahrensstufe zur Rückgewinnung von in diesem enthaltenen Alkan/Alken zugeführt werden. Die Rückgewinnung kann als Ab- oder Adsorption, als Membrantrennung oder Rektifikation durchgeführt werden. In dem Strom enthaltener Wasserstoff kann beispielsweise durch Druckwechseladsorption wieder gewonnen werden. Sowohl das wieder gewonnene Alkan/Alken als auch der wieder gewonnene Wasserstoff können in die Dehydrierung zurückgeführt werden. Es kann auch der gesamte Strom e1 aus dem Verfahren ausgeschleust oder der Verfahrensstufe zur Rückgewinnung von in diesem enthaltenen Alkan/Alken bzw. Wasserstoff zugeführt werden.

Um bessere Stofftrennung zwischen Alken/Alkan einerseits und dem Ester/Säuregemisch andererseits als bei der reinen Entspannung zu erreichen, kann zusätzlich eine Destillation beziehungsweise Rektifikation durchgeführt werden. Typischerweise sind für eine solche Trennung bei einem Rücklaufverhältnis zwischen 0,2 und 1,5 zwischen 10 und 20 theoretische Trennstufen erforderlich. Hier können sowohl Bodenkolonnen, z.B. Glockenbodenkolonnen als auch Packungskolonnen oder Füllkörperkolonnen eingesetzt werden.

Das Produktgemisch d kann auch von einem Druck im Bereich von 20 bis 60 bar auf einen Druck von im Allgemeinen 2 bis 45 bar, beispielsweise 10 bis 40 bar, oder, in einer speziellen Variante, 25 bis 32 bar entspannt werden. Die nach der Entspannung vorliegenden Ströme e1 und e2 können wie nachstehend beschrieben unter Verwendung von zwei Kolonnen K1 und K2 aufgearbeitet werden.

Es können mehrere Varianten dieser Aufarbeitung durchgeführt werden. Die Varianten I zeichnen sich dadurch aus, dass die C3-Komponenten (Propan und Propen) "scharf" abgetrennt werden, wobei nur sehr wenig Propan und Propen im Sumpfaustrag (entspricht Strom e2) enthalten sind (beispielsweise ca. 100 - 1000 Massen-ppm). Vorteil dieser Varianten ist, dass überhaupt keine oder nur sehr wenig C3-Kohlenwasserstoffe in die nachfolgenden Stufen F) und G) gelangen. Werden die C3-Komponenten nicht scharf abgetrennt, wie es der weiter unten beschriebenen Variante II entspricht, so kann dies in den Stufen F) und G) einen höheren verfahrenstechnischen Aufwand mit sich bringen.

Gemäß einer Variante la wird der nach der Entspannung vorliegende Gasstrom e1 einer ersten Kolonne K1 zugeführt. Der Druck in dieser Kolonne ist gleich oder nur wenig geringer als der Druck des Zulaufstroms. Die Kolonne K1 ist bevorzugt der reine Verstärkungsteil einer herkömmlichen Kolonne, d.h. diese besitzt keinen Verdampfer, aber einen Kondensator, und der Zulauf befindet sich bevorzugt unten an der Kolonne. Der nach der Entspannung vorliegende Flüssigstrom e2 wird der zweiten Kolonne K2 zugeführt. Der Druck der Kolonne E2 liegt deutlich unterhalb des Drucks der Kolonne K1. Typische Drücke betragen beispielsweise für K1 = 30 bar (alle Drücke absolut) und für K2 = 1,5 bar. Im Allgemeinen beträgt der Druck in K1 10 bis 40 bar, bevorzugt 25 bis 32 bar, und in K2 1 bis 5 bar, bevorzugt 1,3 bis 2 bar. Der Sumpfabzug der Kolonne K1 wird ebenfalls der Kolonne K2 zugeführt. Die beiden Kopfabzugsströme der Kolonnen K1 und K2 entsprechen dem Strom e1 und können wie oben beschrieben weiterverwendet bzw. weiter aufgearbeitet werden. Dabei wird der Kopfabzug der zweiten Kolonne K2 bevorzugt direkt in die Dehydrierung zurückgeführt, wobei gegebenenfalls ein Verdichter eingesetzt wird.

Gemäß einer weiteren Variante Ib wird wie unter Variante la beschrieben gearbeitet, nur liegt hier der Druck der Kolonne K2 höher (typische Werte beispielsweise K1 = 30 bar, K2 = 5 bar). Im Allgemeinen beträgt der Druck in K1 10 bis 40 bar, bevorzugt 25 bis 32 bar, und in K2 2,5 bis 7 bar, bevorzugt 4 bis 6 bar. Diese Variante hat den Vorteil, dass kein Verdichter benötigt wird.

Gemäß einer weiteren Variante Ic wird wie unter Variante la beschrieben gearbeitet, mit dem Unterschied, dass die Kondensatortemperatur der Kolonne K2 höher liegt (beispielsweise ca. 40°C). Im Allgemeinen beträgt diese Temperatur 30 bis 50°C, bevorzugt 37 bis 45°C. Dadurch enthält der Kopfabzugsstrom der Kolonne K2 noch große Anteile an Alkylester und kann folglich nicht direkt zur Dehydrierung zurückgeführt werden. Deshalb wird dieser Strom auf den Druck der Kolonne K1 komprimiert und der Kolonne K1 zugeführt. Bei dieser Variante entspricht allein der Kopfabzugsstrom der Kolonnen K1 dem Strom e1 und kann wie oben beschrieben weiter verwendet bzw. weiter aufgearbeitet werden. Im Allgemeinen beträgt der Druck in K1 10 bis 40 bar, bevorzugt 25 bis 32 bar, und in K2 1 bis 5 bar, bevorzugt 1,3 bis 2 bar.

Gemäß einer weiteren Variante II wird wie unter Variante Ib beschrieben gearbeitet, jedoch werden größerer Mengen an C3-Komponenten (Propan und Propen) im Sumpfaustrag der Kolonne K2 (beispielsweise ca. 1- 2 Massen-%) zugelassen, so dass die Sumpftemperatur auf ca. 100-110°C begrenzt wird und erhöhte Werkstoffanforderungen an Verdampfer und Sumpfteil der Kolonne K2 nicht resultieren. Der Druck in der Kolonne K1 beträgt im Allgemeinen 10 bis 40 bar, bevorzugt 25 bis 35 bar, der Druck in der Kolonne K2 beträgt im Allgemeinen 2,5 bis 7 bar, bevorzugt 4 bis 6 bar.

In gleicher Weise kann optional auch der Rückführstrom d2 behandelt werden, falls dieser noch größere Mengen an Ester enthält. Auch hier ist eine destillative Reinigung des Stromes bevorzugt. Diese kann in einer separaten Kolonne erfolgen, oder alternativ gemeinsam mit der Behandlung des Stromes d in einer gemeinsamen Kolonne. Es kann sich eine Feinreinigung durch Adsorption, Absorption, Gaswäsche oder katalytische Reinigungsstufen anschließen.

Das den Alkylester (V) enthaltende Produktgemisch e2 (bestehend im Wesentlichen aus dem Alkylester (V) und der organischen Säure (IV), beispielsweise aus Isopropylacetat und Essigsäure) kann weiter aufgearbeitet werden, bevor der Strom e2 in die Verfahrensstufe F) geführt wird. Dabei kann in einer Destillationskolonne die organische Säure (IV), beispielsweise Essigsäure, vom Alkylester (V), beispielsweise Isopropylacetat, abgetrennt werden. Die organische Säure (IV), beispielsweise Essigsäure, kann als Sumpfprodukt gewonnen und in die Esterbildungsstufe zurückgeführt werden. Der Alkylester (V), beispielsweise Isopropylacetat, kann als Kopfprodukt gewonnen werden und wird weiter in die Verfahrensstufe F) geführt. Beispiele für geeignete Prozessparameter im Falle der Trennung von Essigsäure und Isopropylacetat sind ein Druck von bis zu 2 bar (alle Drücke absolut) und ein Rücklaufverhältnis von 0 bis 3. Durch die Abtrennung der organischen Säure wird das Reaktionsgleichgewichts im Hydrolysereaktor der Verfahrensstufe G) positiv beeinflusst. Dadurch kann ein höherer Hydrolyseumsatz ermöglicht werden mit der zusätzlichen Folge, dass die Rückführströme (Wasser = Strom h3; Isopropylacetat = Strom i1) stark reduziert werden. Infolge der deutlich kleineren Rückführströme verringern sich Investitions- und Energiebedarf beispielsweise der nachfolgend beschriebenen Apparate (2), (3), (4) und (5). Durch die beschriebene Abtrennung der organischen Säure (IV) vor Durchführung der Hydrolysestufe F) werden Additionsstufe D) und Hydrolysestufe F) zusätzlich entkoppelt.

In einer Verfahrensstufe F) wird das den Alkylester (V) enthaltende Produktgemisch e2 in einer Esterspaltungszone mit Wasser zu einem Produktgemisch f enthaltend das Alkanol (I) (Isopropanol beziehungsweise 2-Butanol) und die organische Säure (IV) umgesetzt. In einer Verfahrensstufe G) wird aus dem Produktgemisch f das Alkanol (I) abgetrennt und die organische Säure (IV) zurückgewonnen. Die organische Säure (IV) wird im Allgemeinen in die Esterbildungsstufe D) zurückgeführt oder sie wird als Absorptionsmittel in die Absorptionsstufe zur Abtrennung von Alken und Alkan aus der Gasphase c3 geführt.

Das Produktgemisch f kann dabei in einen Strom g1 enthaltend die Säure und einen Strom g2 enthaltend das Alkanol (I) sowie den Alkylester (V) aufgetrennt werden. Aus dem Alkanolstrom g2 kann der Alkylester destillativ abgetrennt und in die Esterspaltungszone zurückgeführt werden. Die Abtrennung des Alkanols (I) aus dem Produktgemisch f kann in Abhängigkeit der Phasengleichgewichte durch einfache Destillation oder durch Azeotrop-Rektifikation unter Einsatz von Schleppmitteln (z.B. Benzol, Cyclohexan oder Diisopropylether im Falle von Isopropanol), durch Extraktivdestillation unter Einsatz eines Extraktionsmittels (z.B. von ionischen Flüssigkeiten oder Essigsäure) sowie durch Membranverfahren (Pervaporation oder Dampfpermeation) erfolgen.

Die Esterspaltung kann sowohl homogen oder heterogen katalysiert durchgeführt werden. Für die Esterspaltung durch Hydrolyse eignen sich grundsätzlich die oben beschriebenen Katalysatoren, die auch bei der Esterbildungsreaktion eingesetzt werden. Bevorzugte heterogene Katalysatoren sind lonenaustauscherharze. Bevorzugte homogene Katalysatoren sind Schwefelsäure oder Heteropolysäuren.

Die Esterspaltungszone kann als Reaktor oder als Reaktivdestillationskolonne ausgestaltet sein. Auch eine Kombination aus Reaktor und Reaktivdestillationskolonne ist möglich.

Vorzugsweise wird die Esterspaltung mit einer unterstöchiometrischen Menge an Wasser durchgeführt. Dadurch wird die organische Säure (IV) im Wesentlichen in konzentrierter Form zurückgewonnen und muss vor Rückführung in die Esterbildungsstufe D) nicht weiter aufkonzentriert werden.

Esterspaltung und Destillation können in getrennten Verfahrensschritten durchgeführt werden. In einer Variante des erfindungsgemäßen Verfahrens wird das den Alkylester (V) enthaltende Gemisch e2 in einem Esterspaltungsreaktor mit Wasser zu einem Produktgemisch f enthaltend das Alkanol (I), die organische Säure (IV), den Alkylester (V) und Wasser umgesetzt und dieses Gemisch anschließend in mindestens 2 hintereinander geschalteten Destillationskolonnen, gegebenenfalls in Verbindung mit einer Trennwandkolonne, aufgetrennt.

Der Esterspaltungsreaktor kann im Falle der heterogen katalysierten Esterspaltung als Festbett-, Rieselbett-, Wirbelschicht- oder Suspensionsreaktor ausgestaltet sein. Im Fall des Wirbelschichtreaktors kann der Katalysator gezielt am Lockerungspunkt betrieben werden (so genannte Schwebebettfahrweise). Im Falle der homogen katalysierten Esterspaltung kann der Reaktor zum Beispiel als Rührkesselreaktor oder Rohrreaktor ausgestaltet sein. Wird die Esterspaltung homogen durchgeführt, so wird der Katalysator im Allgemeinen mit der organischen Säure im Verfahrensschritt G) abgetrennt, im Allgemeinen über den Sumpfabzugsstrom der ersten Destillationskolonne. In diesem Fall kann vor der Rückführung der organischen Säure in die Esterbildungsstufe D) der Katalysator abgetrennt werden, und der abgetrennte Katalysator in den Esterspaltungsreaktor zurückgeführt werden. Die Abtrennung des Katalysators kann thermisch in einem Verdampfer oder in einer mehrstufigen Destillationskolonne sowie in einem Phasenscheider oder aber durch Kombination von Phasenseparation und thermischer Trennung erfolgen. Wird für die Esterspaltung der gleiche homogene Katalysator wie für die Esterbildung eingesetzt, so kann auf eine gesonderte Abtrennung des Katalysators verzichtet werden. Für diese Verfahrensvariante sind Heteropolysäuren oder Schwefelsäure besonders geeignet.

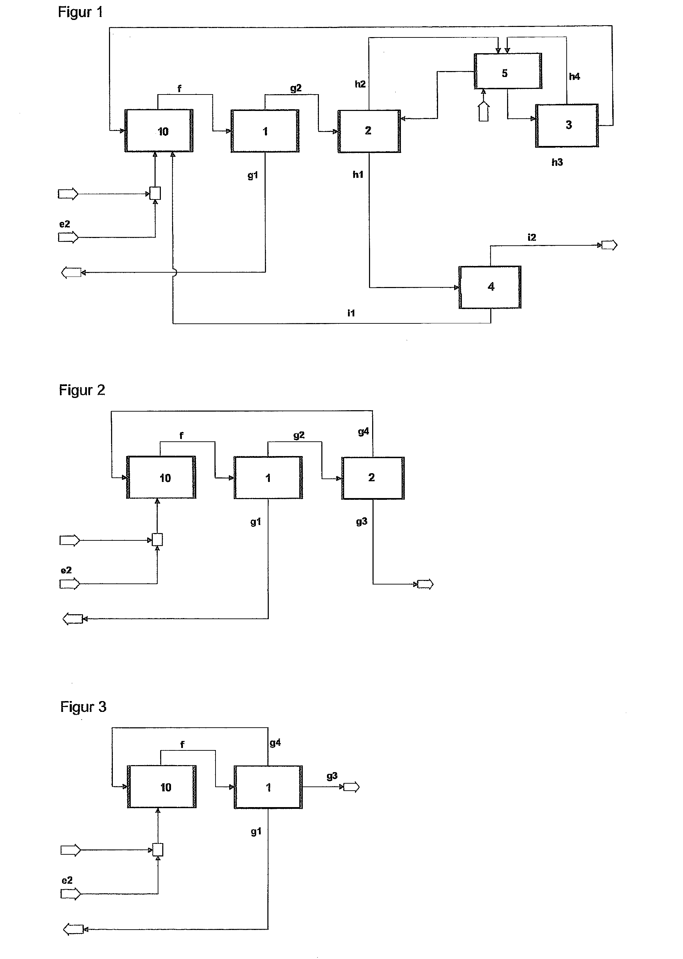

Beispielsweise wird in einer ersten speziellen Ausführungsform des erfindungsgemäßen Verfahrens als organische Säure Essigsäure eingesetzt und ein Isopropylacetat enthaltendes Produktgemisch e2 erhalten, wobei

in Schritt F) das Isopropylacetat enthaltende Produktgemisch e2 in einem Esterspaltungsreaktor mit Wasser zu einem Produktgemisch f enthaltend Isopropanol, Essigsäure, Isopropylacetat und Wasser umgesetzt wird, und

in Schritt G) das Produktgemisch f in einer ersten Destillationskolonne (1) in einen Strom g1 im Wesentlichen bestehend aus Essigsäure und einen Strom g2 enthaltend Isopropylacetat, Isopropanol und Wasser aufgetrennt, und der Strom g2 in einer zweiten Destillationskolonne (2) in einen Strom h1 enthaltend Isopropylacetat und Isopropanol und einen Strom h2 im Wesentlichen bestehend aus Isopropylacetat, Isopropanol und Wasser aufgetrennt wird. Der Strom g1 kann in die Esterbildungszone zurückgeführt werden. Diese Variante ist in Figur 1 dargestellt.

In einer speziellen Variante kann die Auftrennung des Produktgemischs f in einen im Wesentlichen aus Essigsäure bestehenden Strom g1 und einen Isopropylacetat, I-sopropanol und Wasser enthaltenden Strom g2 mit einem zusätzlichen Hydrolyseschritt kombiniert werden. Dazu kann die Destillationskolonne (1) mit einem Seitenreaktor, der den Hydrolysekatalysator enthält, ausgerüstet werden, oder die Destillationskolonne (1) kann als Reaktivdestillationskolonne ausgeführt werden, indem mindestens ein Kolonnenabschnitt mit Böden oder Packungen ausgestattet wird, welche den Hydrolysekatalysator enthalten, oder es kann nach der Destillationskolonne (1) ein Teilstrom des Stroms g2 über einen den Hydrolysekatalysator enthaltenden Reaktor geführt und in die Destillationskolonne (1) rezykliert werden. Die Prozessparameter für den zusätzlichen Hydrolysereaktor sind beispielsweise ein Druck von 2 bis 10 bar und eine Temperatur von 60 bis 130°C.

Der zusätzliche Hydrolysereaktor bringt folgende Vorteile mit sich: da der Reaktionsumsatz im Hydrolysereaktor (Verfahrensschritt F) durch das Reaktionsgleichgewicht limitiert wird, befindet sich der Strom f praktisch im Reaktionsgleichgewicht. Durch die Essigsäureabtrennung in Destillationskolonne (1) wird ein weiterer Hydrolyseumsatz möglich, welcher in dem zusätzlichen Hydrolysereaktor realisiert werden kann. Als Folge des zusätzlichen Hydrolyseumsatzes verringern sich die Rückführströme (Wasser = Strom h3; Isopropylacetat = Strom i1) und entsprechend auch Investitionsbedarf und Energiebedarf der nachfolgend beschriebenen Apparate (2), (3), (4) und (5).

Aus dem Strom h1 kann in einer weiteren Kolonne (4) Isopropanol als Destillat abgetrennt und so ein Strom i1, der im Wesentlichen aus Isopropylacetat besteht, gewonnen und in den Esterspaltungsreaktor (10) zurückgeführt werden. Das in Strom g2 enthaltene Wasser kann durch Azeotropdestillation, umfassend zwei Destillationskolonnen (nachfolgend als zweite (2) und dritte Kolonne (3) bezeichnet) und einen Phasenscheider (5), unter Verwendung eines Schleppmittels, beispielsweise Benzol oder Diisopropylether, abgetrennt und als Strom h3 in den Esterspaltungsreaktor (10) zurückgeführt werden. Sofern bei der Esterspaltung Diisoproplyether als Nebenprodukt anfällt, wird als Schleppmittel in der Wasserabtrennung bevorzugt Diisopropylether eingesetzt.

Die Esterspaltung wird im Allgemeinen bei einem Druck von 1 bis 10 bar, bevorzugt 2 bis 5 bar, und einer Temperatur von 50 bis 150°C, bevorzugt 80 bis 120°C, durchgeführt. Bevorzugt wird dabei in Gegenwart von saurem Ionenaustauscher als Katalysator gearbeitet. Wasser kann in stöchiometrischem Unterschuss oder Überschuss eingesetzt werden, im Allgemeinen wird es in stöchiometrischem Unterschuss, bezogen auf Isopropylacetat, eingesetzt, bevorzugt in Mengen von 0,5 bis 0,9 mol Wasser pro mol Isopropylacetat. Bevorzugt erfolgt die Esterspaltungsreaktion in einem Wirbelschichtreaktor in Schwebebettfahrweise.

Das erhaltene Produktgemisch f enthält beispielsweise 5 bis 20 Gew.-% Isopropanol, 10 bis 50 Gew.-% Essigsäure, 30 bis 70 Gew.-% Isopropylacetat und 5 bis 15 Gew.-% Wasser. Der Strom g1, der im Allgemeinen als Sumpfabzugsstrom der ersten Kolonne (1) gewonnen wird, enthält beispielsweise 90 bis 100 Gew.-% Essigsäure und kann daneben noch 0 bis 5 Gew.-% Isopropylacetat und 0 bis 5 Gew.-% Isopropanol enthalten. Der Strom g2, der im Allgemeinen als Kopfabzugsstrom der ersten Kolonne (1) gewonnen wird, enthält beispielsweise 40 bis 70 Gew.-% Isopropylacetat, 10 bis 80 Gew.-% Isopropanol und 0 bis 20 Gew.-% Wasser. Der Strom h1, der im Allgemeinen als Sumpfabzugsstrom der zweiten Kolonne (2) gewonnen wird, enthält beispielsweise 60 bis 90 Gew.-% Isopropylacetat und 5 bis 30 Gew.-% Isopropanol und 0 bis 5 Gew.-% Essigsäure, und der Strom h2, der im Allgemeinen als Kopfabzugsstrom der zweiten Kolonne (2) gewonnen wird, enthält beispielsweise 30 bis 60 Gew.-% Isopropylacetat, 5 bis 15 Gew.-% Wasser und 10 bis 30 Gew.-% Isopropanol. Daneben kann der Strom h2 noch bis zu 50 Gew.-% des Schleppmittels sowie Spuren von Essigsäure enthalten.

Der als Sumpfabzugsstrom der dritten Kolonne (3) gewonnene Strom h3 enthält vorzugsweise mindestens 95 Gew.-% Wasser, und der Strom h4, der im Allgemeinen als Kopfabzugsstrom der dritten Kolonne (3) gewonnen wird, enthält beispielsweise 5 bis 15 Gew.-% Isopropylacetat, 15 bis 50 Gew.-% Wasser und 30 bis 70 Gew.-% aus Isopropanol. Daneben kann der Strom h4 noch bis zu 5 Gew.-% Schleppmittel und Spuren von Essigsäure enthalten. Der Strom i1, der im Allgemeinen als Sumpfabzugsstrom einer weiteren (vierten) Kolonne (4) gewonnen wird, enthält vorzugsweise mindestens 90 Gew.-% Isopropylacetat, bis zu 5 Gew.% Essigsäure und bis zu 5 Gew.-% Isopropanol, und der Strom i2, der im Allgemeinen als Kopfabzugsstrom der vierten Kolonne (4) gewonnen wird, enthält vorzugsweise mindestens 98 Gew.-% Isopropanol. Daneben kann der Strom i2 noch bis zu 2 Gew.-% Isopropylacetat und Spuren von Schleppmittel und Wasser enthalten.

Im Phasenscheider (5) werden die Kopfabzugsströme h4 und h2 unter Zugabe eines Schleppmittels (Benzol) in eine wässrige und eine organische Phase getrennt. Die wässrige Phase wird als Rücklauf auf die dritte Kolonne (3) gegeben und enthält vorzugsweise mindestens 70 Gew.-% Wasser, bis zu 10 Gew.% Isopropylacetat, bis zu 20 Gew.% Isopropanol und Spuren von Schleppmittel. Die organische Phase wird als Rücklauf auf die zweite Kolonne (2) gegeben und enthält vorzugsweise mindestens 40 Gew.-% Isopropylacetat, bis zu 10 Gew.% Wasser, 10 bis 40 Gew.% Isopropanol und 10 bis 50 Gew.% Schleppmittel sowie Spuren von Essigsäure.

Die erste Kolonne (1) weist im Allgemeinen 10 bis 30 theoretische Böden auf und wird bei einem Druck von 0,5 bis 2 bar betrieben. Die zweite Kolonne (2) weist im Allgemeinen 25 bis 50 theoretische Böden auf und wird bei einem Druck von 0,5 bis 2 bar betrieben. Die dritte Kolonne (3) weist im Allgemeinen 1 bis 15 theoretische Böden auf und wird bei einem Druck von 0,5 bis 2 bar betrieben. Die vierte Kolonne (4) weist im Allgemeinen 40 bis 70 theoretische Böden auf und wird bei einem Druck von 5 bis 10 bar betrieben.

In einer zweiten speziellen Ausführungsform des erfindungsgemäßen Verfahrens wird als organische Säure Ameisensäure eingesetzt und ein Isopropylformiat enthaltendes Produktgemisch e2 erhalten, wobei

in Schritt F) das Isopropylformiat enthaltende Produktgemisch e2 in einem Esterspaltungsreaktor (10) mit Wasser zu einem Produktgemisch f enthaltend Isopropanol, Ameisensäure, Isopropylformiat und Wasser umgesetzt wird, und

in Schritt G) das Produktgemisch f in einer ersten Destillationskolonne (1) in einen Strom g1 enthaltend Ameisensäure und Wasser und einen Strom g2 enthaltend Isopropylformiat, Wasser und Isopropanol aufgetrennt wird, und der Strom g2 in einer zweiten Destillationskolonne (2) in einen Strom g4 enthaltend Isopropylformiat, Wasser und Isopropanol und einen Strom g3 im Wesentlichen bestehend aus Isopropanol aufgetrennt wird und der Strom g1 in die Esterbildungszone und der Strom g4 in den Esterspaltungsreaktor (10) zurückgeführt wird. g3 wird im Allgemeinen als Sumpfabzugsstrom oder gasförmiger Seitenabzugsstrom erhalten. Zusätzlich kann aus Strom g1 Wasser von Ameisensäure getrennt werden und somit nur die aufkonzentrierte Ameisensäure zurück in die Esterbildungszone geführt werden, wodurch höhere Raum/Zeit-Ausbeuten erzielt werden und der ameisensäurearme Wasserstrom zurück in den Esterspaltungsreaktor geführt wird. Diese Variante ist in Figur 2 dargestellt.

Die Esterspaltung wird im Allgemeinen bei einem Druck von 1 bis 10 bar, bevorzugt 2 bis 5 bar, und einer Temperatur von 50 bis 150°C, bevorzugt 80 bis 120°C, durchgeführt. Bevorzugt wird dabei in Gegenwart von saurem Ionentauscher als Katalysator gearbeitet. Wasser wird im Allgemeinen in stöchiometrischem Unterschuss, bezogen auf Isopropylformiat, eingesetzt, bevorzugt in Mengen von 0,5 bis 0,9 mol Wasser pro mol Isopropylformiat. Gegebenenfalls kann Wasser auch in stöchiometrischem Überschuss zugegeben werden.

Das Produktgemisch f enthält beispielsweise 10 bis 25 Gew.-% Isopropanol, 15 bis 40 Gew.-% Ameisensäure, 20 bis 60 Gew.-% Isopropylformiat und 5 bis 20 Gew.-% Wasser.

Der Strom g1, der im Allgemeinen als Sumpfabzugsstrom der ersten Kolonne (1) gewonnen wird, enthält im Allgemeinen ein Gemisch aus beispielsweise 80 bis 95 Gew.-% Ameisensäure und 5 bis 20 Gew.-% Wasser. Der Strom g2, der im Allgemeinen als Kopfabzugsstrom der ersten Kolonne (1) gewonnen wird, enthält beispielsweise 50 bis 80 Gew.-% Isopropylformiat, bis zu 10 Gew.-% Wasser und 20 bis 40 Gew.-% Isopropanol. Der Strom g4, der im Allgemeinen als Kopfabzugsstrom der zweiten Kolonne (2) gewonnen wird, enthält ein Gemisch aus beispielsweise 70 bis 90 Gew.-% Isopropylformiat, bis zu 15 Gew.-% Wasser und 5 bis 15 Gew.-% Isopropanol. Der Strom g3 im Allgemeinen als Sumpfabzugsstrom oder gasförmiger Seitenabzugsstrom der zweiten Kolonne (2) besteht vorzugsweise zu mindestens 98 Gew.-% aus Isopropanol und kann daneben noch Wasser in Mengen bis zu 2 Gew.-% enthalten.

Die erste Kolonne (1) weist im Allgemeinen 30 bis 60 theoretische Böden auf und wird bei einem Druck von 0,5 bis 3 bar betrieben. Die zweite Kolonne (2) weist im Allgemeinen 30 bis 60 theoretische Böden auf und wird bei einem Druck von 0,5 bis 1 bar betrieben.

In einer dritten speziellen Ausführungsform des erfindungsgemäßen Verfahrens wird in Schritt F) das Isopropylformiat enthaltende Produktgemisch e2 in einem Esterspaltungsreaktor (10) mit Wasser zu einem Produktgemisch f enthaltend Isopropanol, Ameisensäure, Isopropylformiat und Wasser umgesetzt und

in Schritt G) das Produktgemisch f diesseits der Trennwand in eine Trennwand-Destillationskolonne (1) geführt in einen Strom g1 enthaltend Ameisensäure und Wasser, einen Destillatstrom g4 enthaltend Isopropylformiat, Wasser und Isopropanol und einen Seitenstrom g3 jenseits der Trennwand im Wesentlichen bestehend aus Isopropanol aufgetrennt und der Sumpfstromstrom g1 in die Esterbildungszone und der Strom g4 in den Esterspaltungsreaktor (10) zurückgeführt. Die Zusammensetzung der gewonnenen Ströme g1, g3 und g4 ist vorzugsweise die gleiche wie bei der Verschaltung von zwei getrennten Kolonnen. Die Trennwandkolonne (1) weist im Allgemeinen 10 bis 30 theoretische Böden in der Vorkolonne und 30 bis 60 theoretische Böden in der Hauptkolonne auf und wird bei einem Druck von 0,5 bis 3 bar betrieben. Diese Variante ist in Figur 3 dargestellt.

Esterspaltung und Destillation können aber auch zum Teil simultan in ein und demselben Verfahrensschritt in einer Reaktivdestillationskolonne durchgeführt werden, wobei das durch Esterspaltung gebildete Gemisch gleichzeitig zumindest teilweise destillativ aufgetrennt wird und die organische Säure (gegebenenfalls zusammen mit einem Homogenkatalysator) zurückgewonnen wird. Der Reaktivdestillationskolonne kann ein Vorreaktor vorgeschaltet sein.

Die Reaktivdestillation kann homogen oder heterogen katalysiert durchgeführt werden. Die Reaktivdestillationskolonne kann übliche Einbauten (beispielsweise Packungen, Füllkörper, Böden) enthalten. Heterogenkatalysatoren können in Form katalytischer Einbauten, beispielsweise als katalytische Packungen oder Füllkörper, oder als Suspension vorliegen. Zudem ist eine Unterbringung des Katalysators in außen liegenden Behältern möglich, die von einem Seitenstrom der Reaktionskolonne gespeist werden.

In einer Variante des erfindungsgemäßen Verfahrens wird das den Ester enthaltende Gemisch e2 in einem Esterspaltungs-Vorreaktor mit Wasser zu einem Produktgemisch f enthaltend das Alkanol (I), die organische Säure (IV), den Alkylester (V) und Wasser umgesetzt und aus dem Produktgemisch f in einer Reaktivdestillationskolonne ein Produktgemisch f2 aus dem Alkanol (I) und dem Alkylester (V) und ein Strom f3 aus der organischen Säure (IV), der in die Esterbildungszone zurückgeführt wird, gewonnen und das Produktgemisch f2 in einer nachgeschalteten (weiteren) Destillationskolonne in einen Strom g1 aus dem Alkanol (I) und dem Alkylester (V) und geringen Mengen an Wasser und einen Strom g2 aus dem Alkanol (I) aufgetrennt, wobei der Strom g1 in die Esterspaltungszone, bevorzugt in die Reaktivdestillationskolonne, zurückgeführt wird.

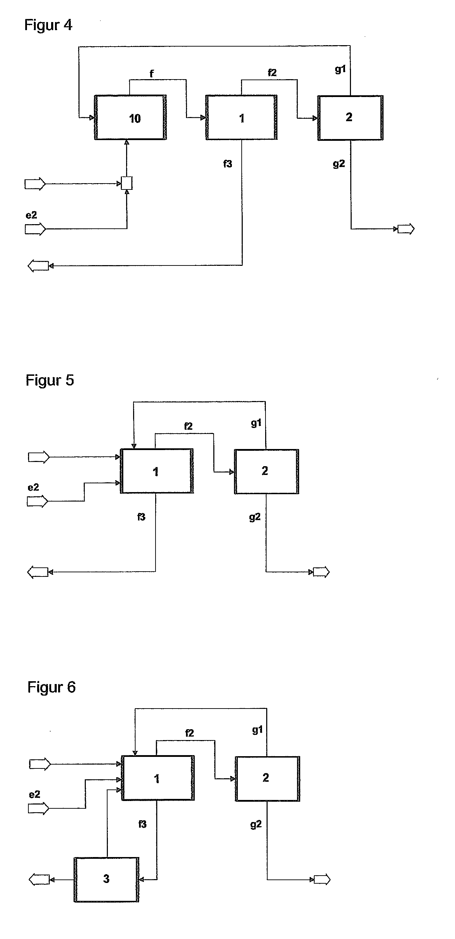

So wird beispielsweise in einer vierten speziellen Ausführungsform des erfindungsgemäßen Verfahrens in Schritt F) das Isopropylacetat enthaltende Produktgemisch e2 in einem Esterspaltungs-Vorreaktor mit Wasser zu einem Produktgemisch f enthaltend Isopropanol, Essigsäure, Isopropylacetat und Wasser umgesetzt, wird aus dem Produktgemisch f in einer Reaktivdestillationskolonne (1) ein Produktgemisch f2 im Wesentlichen bestehend aus Isopropanol, Isopropylacetat und geringen Mengen Wasser und ein Strom f3 im Wesentlichen bestehend aus Essigsäure gewonnen und der Strom f3 in die Esterbildungszone zurückgeführt, und

in Schritt G) das Produktgemisch f2 in einer Destillationskolonne (2) in einen Strom g1 im Wesentlichen bestehend aus Isopropanol, Isopropylacetat und geringen Mengen Wasser und einen Strom g2 im Wesentlichen bestehend aus Isopropanol aufgetrennt. Der Strom g1 kann in den Esterspaltungsreaktor (10) oder bevorzugt in die Reaktivdestillationskolonne (1) zurückgeführt werden. Diese Variante ist in Figur 4 dargestellt.

Das Produktgemisch f, das beispielsweise 5 bis 20 Gew.-% Isopropanol, 10 bis 90 Gew.-% Essigsäure, 10 bis 80 Gew.-% Isopropylacetat und 1 bis 20 Gew.-% Wasser enthält, wird in der Reaktivdestillationskolonne (1) weiter umgesetzt und gleichzeitig in einen Strom f2, welcher beispielsweise 30 bis 80 Gew.-% Isopropanol und 10 bis 70 Gew.-% Isopropylacetat und bis zu 10 Gew.-% Wasser enthält und im Allgemeinen als Kopfabzugsstrom gewonnen wird, und einen Strom f3, der vorzugsweise zu mindestens 90 Gew.-% aus Essigsäure besteht, daneben noch Isopropylacetat und Isopropanol enthalten kann und im Allgemeinen als Sumpfabzugsstrom gewonnen wird, aufgetrennt.

In der Reaktionskolonne können die katalytisch wirkenden Einbauten sowohl unterhalb als auch oberhalb des Feedzulaufes angeordnet werden. Am Kopf und im Sumpf der Kolonne können sich darüber hinaus noch weitere trennwirksame Einbauten befinden. Bevorzugt befindet sich der Feedzulauf unterhalb der Reaktionszone. Die Reaktionskolonne weist im Allgemeinen 30 bis 80 theoretische Böden auf und wird bei einem Druck von 1 bis 6 bar betrieben. Zusätzlich zu dem Feedzulauf f kann Wasser oberhalb und unterhalb der Reaktionszone sowie in die Reaktionszone selbst zugegeben werden.

Der Strom g1, der im Allgemeinen als Kopfabzugsstrom der Destillationskolonne (2) gewonnen wird, enthält beispielsweise 10 bis 50 Gew.-% Isopropanol und 30 bis 90 Gew.-% Isopropylacetat. Der Strom g2, der im Allgemeinen als Sumpfabzugsstrom oder gasförmiger Seitenabzugsstrom der Destillationskolonne (2) gewonnen wird, besteht vorzugsweise zu mindestens 98 Gew.-% aus Isopropanol. Daneben kann er noch Isopropylacetat enthalten, im Allgemeinen in Mengen von bis zu 1 Gew.-%. Die Destillationskolonne (2) weist im Allgemeinen 10 bis 50 theoretische Böden auf und wird bei einem Druck von 0,01 bis 1 bar betrieben.

Der der Reaktivdestillationskolonne (1) vorgeschaltete Esterspaltungsreaktor (10) kann auch entfallen. Der Vorteil des Vorreaktors ist bei der heterogen katalysierten Reaktivdestillation, dass hier die Katalysatormenge in der Kolonne reduziert werden kann und zudem der Vorreaktor als Schutzbett vor Katalysatorgiften wirkt.

So wird in einer fünften speziellen Ausführungsform des erfindungsgemäßen Verfahrens in Schritt F) das Isopropylacetat enthaltende Produktgemisch e2 mit Wasser direkt in eine Reaktivdestillationskolonne (1) eingespeist und aus dem Produktgemisch e2 in der Reaktivdestillationskolonne (1) ein Produktgemisch f2 im Wesentlichen bestehend aus Isopropanol, Isopropylacetat und geringen Mengen Wasser und ein Strom f3 im Wesentlichen bestehend aus Essigsäure gewonnen und der Strom f3 in die Esterbildungszone zurückgeführt, und

in Schritt G) das Produktgemisch f2 in einer Destillationskolonne (2) in einen Strom g1 im Wesentlichen bestehend aus Isopropanol, Isopropylacetat und geringen Mengen Wasser und einen Strom g2 im Wesentlichen bestehend aus Isopropanol aufgetrennt und der Strom g1 in die Reaktivdestillationskolonne (1) zurückgeführt. Diese Variante ist in Figur 5 dargestellt.

Die Zusammensetzung der Ströme f2, f3, g1 und g2 entspricht im Wesentlichen der Fahrweise mit vorgeschaltetem Esterspaltungsreaktor.

Neben dem Einsatz eines heterogenen Katalysators zur Esterspaltung kann ebenfalls ein homogener Katalysator verwendet werden.

So wird in einer sechsten speziellen Ausführungsform des erfindungsgemäßen Verfahrens in Schritt F) das Isopropylacetat enthaltende Produktgemisch e2 mit Wasser in eine Reaktivdestillationskolonne (1) eingespeist und aus dem Produktgemisch e2 in der Reaktivdestillationskolonne (1) ein Produktgemisch f2 im Wesentlichen bestehend aus Isopropanol, geringen Mengen Wasser und Isopropylacetat und ein Strom f3 im Wesentlichen bestehend aus Essigsäure und dem homogenen Katalysator gewonnen und in die Esterbildungszone zurückgeführt, und

in Schritt G) das Produktgemisch f2 in einer Destillationskolonne (2) in einen Strom g1 im Wesentlichen bestehend aus Isopropylacetat, Wasser und Isopropanol und einen Strom g2 im Wesentlichen bestehend aus Isopropanol aufgetrennt und der Strom g1 in die Reaktivdestillationskolonne zurückgeführt. Diese Variante ist in Figur 6 dargestellt.

Die Abtrennung des Katalysators aus Strom f3 kann thermisch in einem Verdampfer oder einer mehrstufigen Destillationskolonne sowie in einem Phasenscheider oder der Kombination aus Phasenscheider und thermischer Abtrennung erfolgen. Der aufkonzentrierte Katalysatorstrom wird in den Verstärkungsteil der Reaktionskolonne und die aufkonzentrierte Essigsäure zurück in die Esterbildungsstufe zurückgeführt.

Die Zusammensetzung der Ströme f2, g1 und g2 entspricht im Wesentlichen der Fahrweise mit heterogen katalysierter Reaktivdestillation. Der Strom f3, bevorzugt bestehend aus Essigsäure, kann bis zu 50 Gew.-% Katalysator enthalten.

In der Reaktionskolonne (1) befinden sich konventionelle Einbauten zur Destillation. Bevorzugt befinden sich in der Reaktionszone Kolonnenböden, z.B. Kaminböden, die die Einstellung definierter Verweilzeiten erlauben. Neben internen Kolonneneinbauten können definierte Verweilzeiten zudem in einem externen an die Kolonne angeschlossenen Behälter realisiert werden, indem mittels eines Seitenabzuges ein Strom aus der Kolonne abgezogen, durch den Behälter geleitet und zurück in die Kolonne geführt wird. Die Reaktionskolonne weist im Allgemeinen 30 bis 100 theoretische Böden auf und wird bei einem Druck von 1 bis 6 bar betrieben. Zusätzlich zu dem Feedzulauf e2 kann Wasser in die Reaktionszone zugegeben werden.

In den oben beschriebenen vierten, fünften und sechsten speziellen Verfahrensvarianten ist es bevorzugt, in Schritt F) Wasser in - bezogen auf Isopropylacetat - stöchiometrischem Unterschuss einzusetzen. Hierdurch wird, da Wasser in der Reaktionskolonne nahezu vollständig umgesetzt wird, die Ausbildung von wässrig-organischen Azeotropen (wie Isopropanol-Wasser, Isopropanol-Isopropylacetat-Wasser) vermieden.

Auch im Falle von Isopropylformiat kann die Esterspaltung als Reaktivdestillation (mit und ohne Vorreaktor) durchgeführt werden.

So wird in einer siebten speziellen Ausführungsform des erfindungsgemäßen Verfahrens in Schritt F) das Isopropylformiat enthaltende Produktgemisch e2 in einem Esterspaltungsreaktor (10) mit Wasser zu einem Produktgemisch f enthaltend Isopropanol, Ameisensäure, Isopropylformiat und Wasser umgesetzt und aus dem Produktgemisch f in einer Reaktivdestillationskolonne (1) ein Produktgemisch f2 im Wesentlichen bestehend aus Isopropanol und Isopropylformiat und ein Strom f3 im Wesentlichen bestehend aus Ameisensäure und Wasser gewonnen und der Strom f3 in die Esterbildungszone zurückgeführt, und

in Schritt G) das Produktgemisch f2 in einer Destillationskolonne (2) in einen Strom g1 im Wesentlichen bestehend aus Isopropanol und Isopropylformiat und einen Strom g2 im Wesentlichen bestehend aus Isopropanol aufgetrennt und der Strom g1 in den Esterspaltungsreaktor (10) oder bevorzugt in die Reaktivdestillationskolonne (1) zurückgeführt. Diese Variante ist ebenfalls in Figur 4 dargestellt.

Das Produktgemisch f, das beispielsweise 5 bis 15 Gew.-% Isopropanol, 5 bis 50 Gew.-% Ameisensäure, 20 bis 70 Gew.-% Isopropylformiat und 1 bis 15 Gew.-% Wasser enthält, wird in der Reaktivdestillationskolonne (1) weiter umgesetzt und gleichzeitig in einen Strom f2, welcher beispielsweise 10 bis 50 Gew.-% Isopropanol und 30 bis 80 Gew.-% Isopropylformiat enthält und im Allgemeinen als Kopfabzugsstrom gewonnen wird, und einen Strom f3, der beispielsweise 60 bis 85 Gew.-% Ameisensäure und 5 bis 20 Gew.-% Wasser enthält, daneben noch Isopropylformiat enthalten kann und im Allgemeinen als Sumpfabzugsstrom gewonnen wird, aufgetrennt.

Der Strom g1, der im Allgemeinen als Kopfabzugsstrom der Destillationskolonne (2) gewonnen wird, enthält im Allgemeinen ein azeotrop siedendes Gemisch aus beispielsweise 5 bis 30 Gew.-% Isopropanol und 60 bis 95 Gew.-% Isopropylformiat. Der Strom g2, der im Allgemeinen als Sumpfabzugsstrom oder gasförmiger Seitenabzugsstrom im Abtriebsteil der Destillationskolonne (2)gewonnen wird, besteht vorzugsweise zu mindestens 99 Gew.-% aus Isopropanol. Daneben kann er noch Isopropylformiat enthalten, im Allgemeinen in Mengen von bis zu 1 Gew.-%. Die Destillationskolonne (2) weist im Allgemeinen 10 bis 30 theoretische Böden auf und wird bei einem Druck von 0,1 bis 1 bar betrieben.

In den oben beschriebenen siebten speziellen Verfahrensvarianten ist es ebenfalls bevorzugt, in Schritt F) Wasser in - bezogen auf Isopropylformiat - stöchiometrischem Unterschuss einzusetzen. Hierdurch wird, da Wasser in der Reaktionskolonne nahezu vollständig umgesetzt wird, die Ausbildung von wässrig-organischen Azeotropen (wie Isopropanol-Wasser, Isopropanol-Isopropylformiat-Wasser) vermieden.

In der Reaktionskolonne (1) können die katalytisch wirkenden Einbauten sowohl unterhalb als auch oberhalb des Feedzulaufes angeordnet sein. Am Kopf und im Sumpf der Kolonne können sich darüber hinaus noch weitere trennwirksame Einbauten befinden. Bevorzugt befindet sich der Feedzulauf unterhalb der Reaktionszone. Die Reaktionskolonne weist im Allgemeinen 30 bis 80 theoretische Böden auf und wird bei einem Druck von 1 bis 6 bar betrieben.

Der der Reaktivdestillationskolonne (1) vorgeschaltete Esterspaltungsreaktor (10) kann ebenfalls entfallen. Die nachgeschaltete Kolonne (2) und die Reaktionskolonne (1) können auch zu einer reaktiven Trennwandkolonne zusammengefasst werden.

Die Erfindung wird durch die nachstehenden Beispiele näher erläutert.

### Beispiel 1

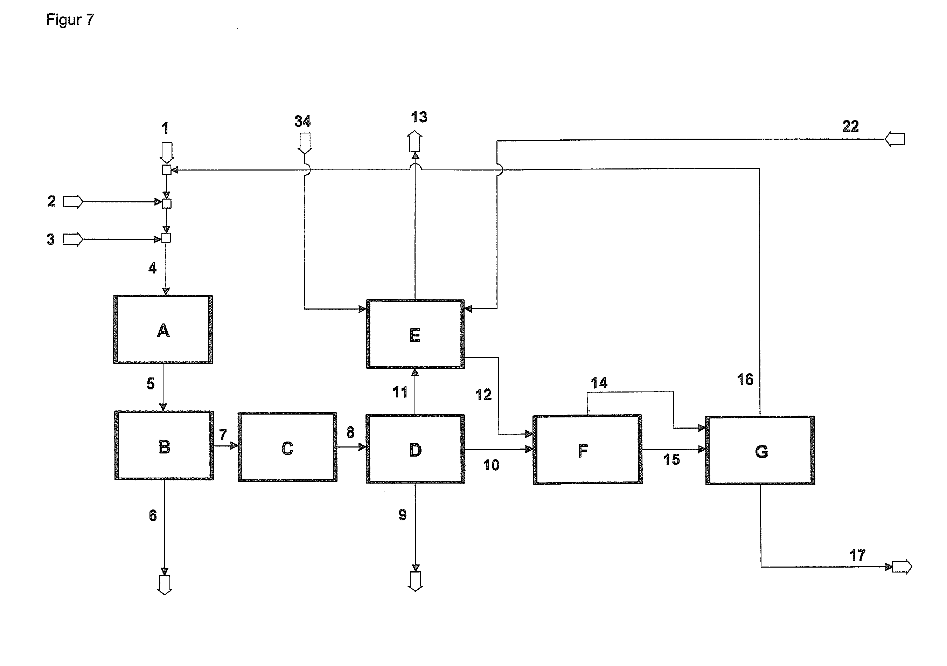

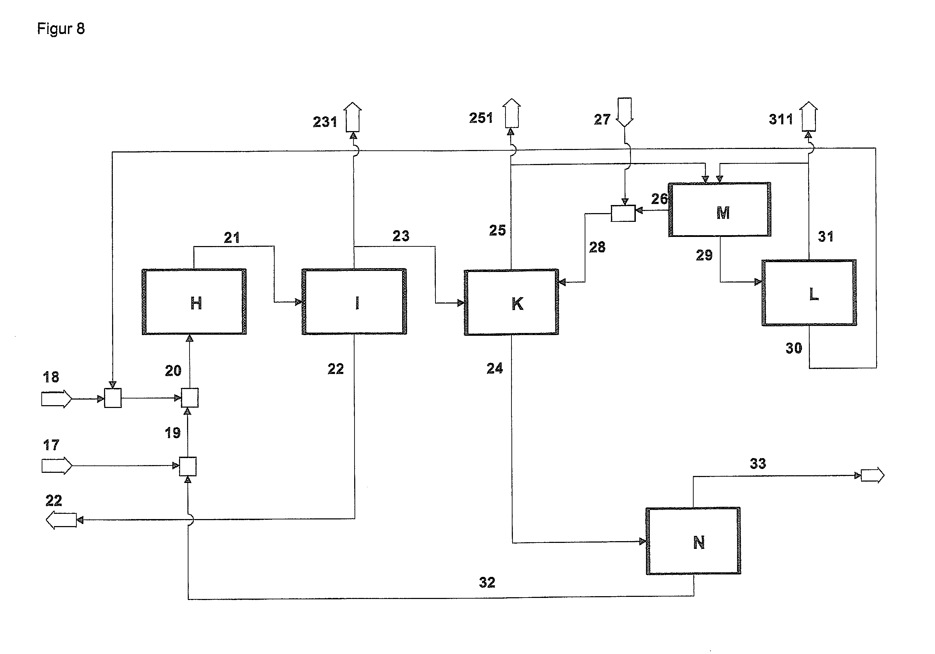

Mit Hilfe des kommerziellen Simulationsprogramms AspenPlus wurde beispielhaft ein integriertes Gesamtverfahren zur Produktion von Isopropanol aus Propan berechnet. Das Verfahren umfasst in diesem Beispiel die Stufen A bis N. Diese sind in den Figuren 7 und 8 mit den zugehörigen Stoffströmen dargestellt. Die berechneten Zusammensetzungen der einzelnen Stoffströme können der Tabelle entnommen werden. Die Simulationsrechnung basiert auf einem Zustandsgleichungsmodell auf Basis von Messungen von Dampf-Flüssig- und Flüssig-Flüssig-Phasengleichgewichten.

Dem Propandehydrierreaktor A wird ein propanreicher, gasförmiger Strom (4) zugeführt. Dieser setzt sich aus dem Rückführstrom (16) vom Kopf der Rektifikationskolonne G, der im wesentlichen Propan enthält, sowie den Zuläufen von Frisch-Propan (1), Wasser (2) und Sauerstoff (3) zusammen. Das den Reaktor A verlassende 590°C heiße Dehydriergasgemisch (5) wird in der Abkühl- und Kondensationsstufe B stufenweise abgekühlt, wobei ein wässriger Kondensatstrom (6) anfällt. Der Produktgasstrom (7) wird der Kompressionsstufe C zugeführt, in der er auf 40 bar verdichtet wird. Der verdichtete Strom (8) wird im Phasenscheider D in drei Teilströme getrennt. Der Gasstrom (11) wird der Absorptionskolonne E, der flüssige, Propan- und Propen-reiche Strom (10) wird dem Esterbildungsreaktor F zugeführt. Außerdem fällt im Phasenscheider D ein wässriger Strom (9) an. In der Absorptionskolonne E wird bevorzugt Propan und Propen in einem Absorptionsmittel gelöst und zusammen mit dem Absorptionsmittel als Strom (12) dem Esterbildungsreaktor F zugeführt. Als Absorptionsmittel wird der vom Sumpfabzug der Rektifikationskolonne I zurückgeführte Essigsäure-reiche Strom (22) eingesetzt, der mit frischer Essigsäure (34) ergänzt wird. Der Gasstrom (13) kann als Abgas ausgeschleust oder aber, gegebenenfalls nach einem zusätzlichen Trennschritt, wieder in den Dehydrierreaktor A zurückgeführt werden. Im Esterbildungsreaktor F werden bei 40 bar und 110°C 90 % des mit den Strömen (12) und (10) zugeführten Propens mit Essigsäure zu Isopropylacetat umgesetzt. Für die Berechnung wird eine Selektivität von 100 % zu Isopropylacetat angenommen. Die beiden Austrittsströme aus dem Esterbildungsreaktor F, der gasförmige Strom (14) und der flüssige Strom (15), werden der Rektifikationskolonne G zugeführt. Der gasförmige Kopfabzugsstrom (16) der Kolonne G wird in den Propandehydrierreaktor A und der flüssige Sumpfabzugsstrom (17) in den Esterspaltungsreaktor H geführt. Neben diesem Strom (17) werden dem Esterspaltungsreaktor H Wasser (18) sowie die beiden Sumpfabzugströme (30) und (32) der Rektifikationskolonnen L beziehungsweise N zugeführt. Im Esterspaltungsreaktor H wird bei 5 bar und 100°C 20 % Isopropylacetat bei einer angenommenen Selektivität von 100 % zu Isopropanol umgesetzt. Der Austrittsstrom (21) aus dem Esterspaltungsreaktor H wird der nachfolgenden Rektifikationskolonne I zugeführt. Der essigsäurereiche Sumpfabzugsstrom (22) der Kolonne I wird in die Absorptionskolonne E zurückgeführt. Am Kopfkondensator der Rektifikationskolonne I wird ein Abgasstrom (231) aus unter diesen Bedingungen nicht kondensierbaren Gasen abgezogen und der verbleibende Kopfabzugsstrom (23) in die Strippkolonne K geführt. In den Strippkolonnen K und L sowie dem Phasenscheider M wird eine Heteroazeotroprektifikationsstufe mit Benzol als Schleppmittel durchgeführt. Jeweils an den oberen Enden der beiden Kolonnen K und L wird ein Abgasstrom aus unter diesen Bedingungen nicht kondensierbaren Gasen abgezogen (Ströme 251 und 311). Die beiden verbleibenden Kopfabzugsströme (25) beziehungsweise (31) werden in den Phasenscheider M geführt. Aus dem Phasenscheider M wird die obere flüssige Phase (26) mit dem Zulaufstrom an frischem Benzol (27) gemeinsam als Strom (28) auf den Kopf der Strippkolonne K und die untere flüssige Phase (29) auf den Kopf der Strippkolonne L geführt. Der im Wesentlichen Wasser enthaltende Sumpfabzugsstrom (30) der Strippkolonne L wird zum Esterspaltungsreaktor H zurückgeführt. Der Sumpfabzugsstrom (24) der Strippkolonne K wird der Rektifikationskolonne N zugeführt. Der Sumpfabzugsstrom (32) der Kolonne N, der im Wesentlichen Isopropylacetat enthält, wird zum Esterespaltungsreaktor H zurückgeführt. Am Kopf der Rektifikationskolonne N wird der gewünschte Produktstrom (33) mit einem Gehalt von 99,7 Massen-% Isopropanol erhalten.

In der nachstehenden Tabelle 1 sind die einzelnen Stufen A - N nochmals zusammengefasst. Die Zusammensetzung der Ströme gibt Tabelle 2 wieder.

**Tabelle 1:**

| Apparateliste | |
|---|---|
| **Stufe** | **Beschreibung** |
| **A** | Dehydrierreaktor; 2,5 bar; 590°C; Umsatz Propan: 35 %; Selektivität zu Propen: 95 % |
| **B** | Abkühl- und Kondensationsstufe; 2,4 bar; Endtemperatur 60°C |
| **C** | Kompressionsstufe; Enddruck 40 bar |
| **D** | Phasenscheider; 40 bar; 60°C |
| **E** | Absorptionskolonne; 40 bar; Zulauf Absorptionsmittel bei 40°C; 30 theoretische Stufen |
| **F** | Esterbildungsreaktor; 40 bar; 110°C; Umsatz Propen: 90 %; Selektivität zu Isopropylacetat: 100 % |
| **G** | Rektifikationskolonne; 5 bar; 13 theoretische Stufen, Rücklaufverhältnis: 0,6 |
| **H** | Esterspaltungsreaktor; 5 bar, 100°C; Umsatz Isopropylacetat: 20 %; Selektivität zu Isopropanol: 100% |
| **I** | Rektifikationskolonne; 1 bar; 21 theoretische Stufen, Rücklaufverhältnis: 0,5 |
| **K** | Strippkolonne; 1 bar; 35 theoretische Stufen |
| **L** | Strippkolonne; 1 bar; 3 theoretische Stufen |
| **M** | Phasenscheider; 1 bar; 40°C |
| **N** | Rektifikationskolonne; 7 bar; 60 theoretische Stufen, Rücklaufverhältnis: 6,6 |

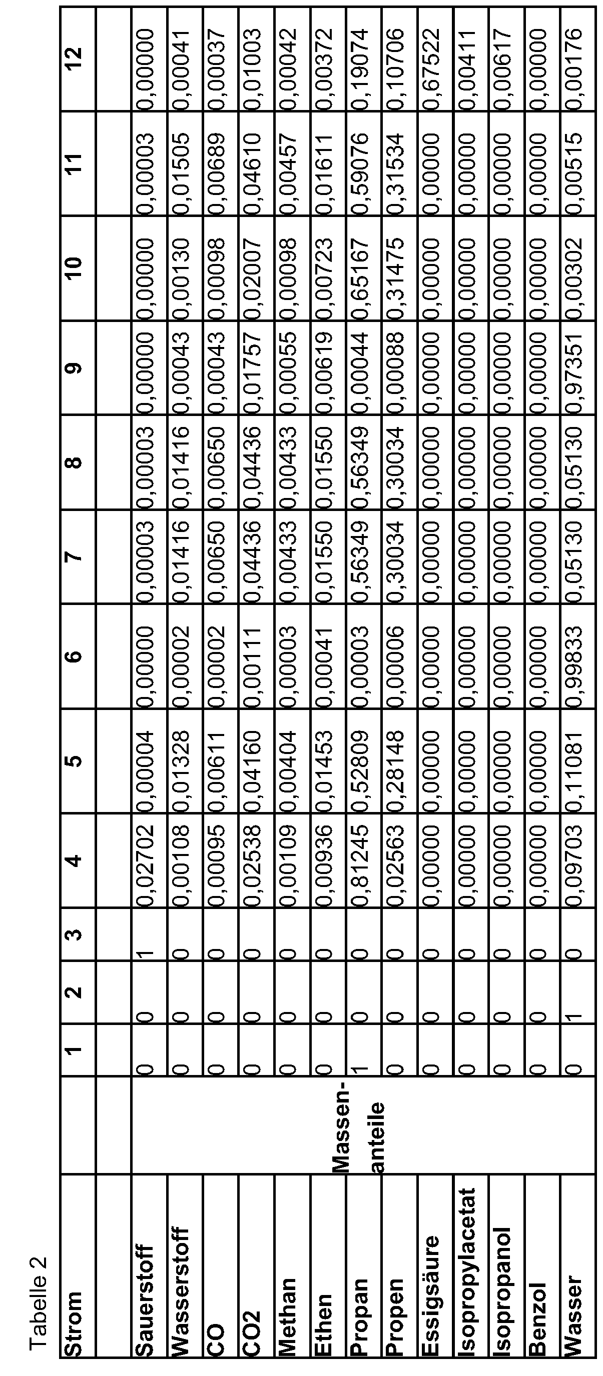

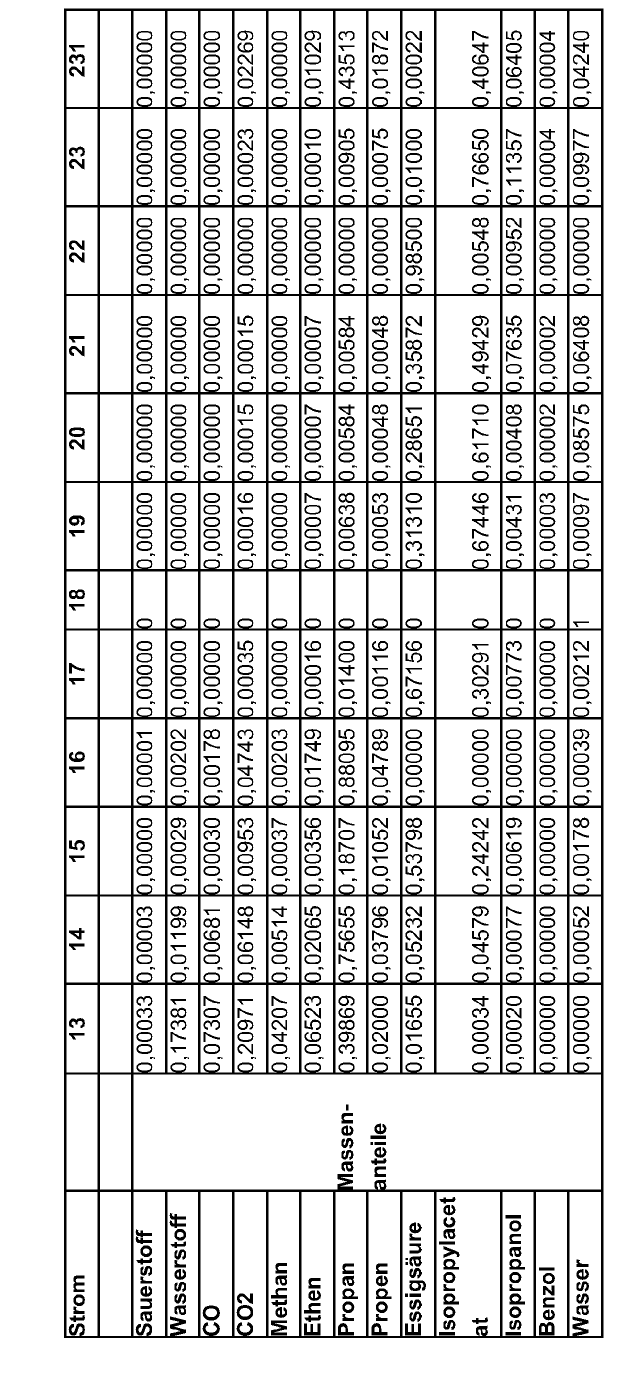

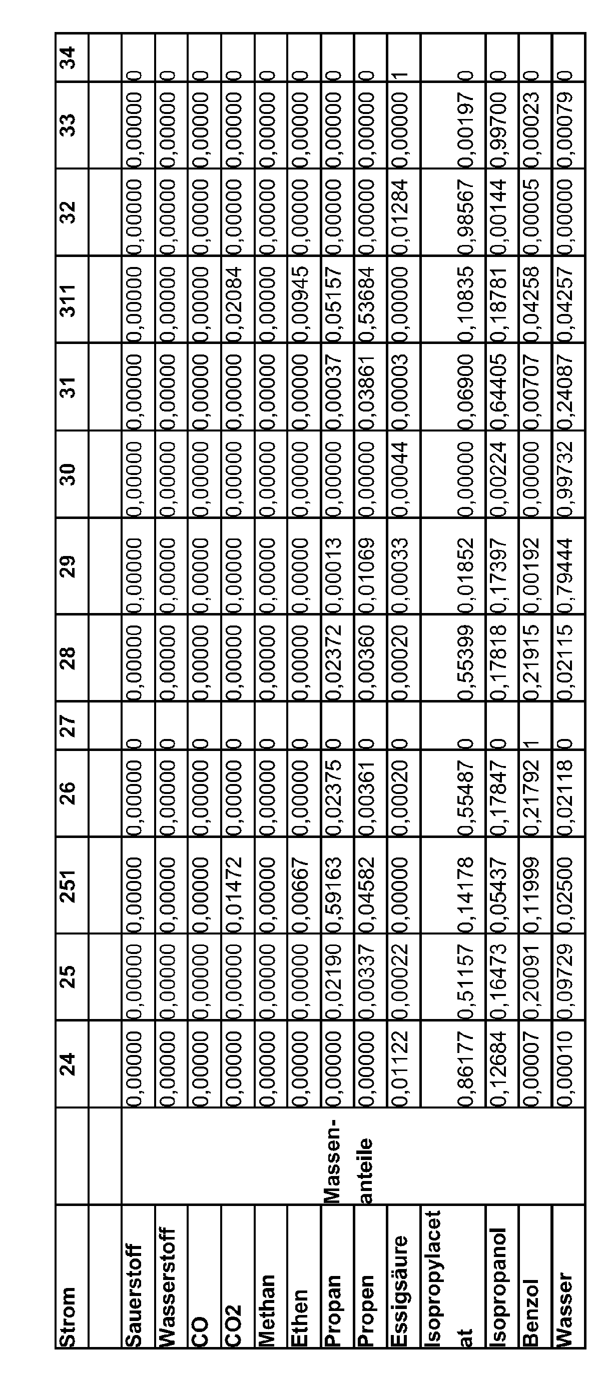

### Beispiel 2

In einem kontinuierlich betriebenen Autoklav mit einem Reaktionsvolumen von 200 ml werden bei 110°C und 40 bar 16,9 g Propylen, 34,4 g Propan und 116,7 g Essigsäure pro Stunde in Anwesenheit von 15,7 g "Amberlyst 35 wet" als Katalysator zur Reaktion gebracht. Der Austragsstrom wird entspannt und die flüssige und gasförmige Phase getrennt voneinander per GC analysiert. Man erhält bei einem Propylenumsatz von 90 % Isopropylacetat mit einer Selektivität von über 96 %.

## Patentansprüche

1. Verfahren zur Herstellung von Isopropanol aus Propan mit den Schritten:
A) ein Propan enthaltender Einsatzgasstrom a wird bereitgestellt;
B) der Propan enthaltende Einsatzgasstrom a wird in eine Dehydrierzone eingespeist und Propan wird einer Dehydrierung zum Propen unterworfen, wobei ein Produktgasstrom b enthaltend Propen, nicht umgesetztes Propan, gegebenenfalls Hochsieder, Wasserdampf, Wasserstoff und Leichtsieder erhalten wird;
C) der Produktgasstrom b wird zumindest komprimiert, optional wird der Produktgasstrom b in eine wässrige Phase c1, eine Propen, Propan und gegebenenfalls Hochsieder enthaltende Phase c2 und eine Gasphase c3 enthaltend Wasserstoff und Leichtsieder aufgetrennt;
D) der Produktgasstrom b beziehungsweise die Propen und Propan enthaltende Phase c2 wird in einer Esterbildungszone mit Essigsäure umgesetzt, wobei ein Produktgemisch d enthaltend Isopropylacetat und nicht umgesetztes Propan erhalten wird;
E) von dem Produktgemisch d wird ein Propan enthaltender Gasstrom e1 abgetrennt, der gegebenenfalls in die Dehydrierzone zurückgeführt wird, und ein Isopropylacetat enthaltendes Produktgemisch e2 gewonnen;
F) das Isopropylacetat enthaltende Produktgemisch e2 wird mit Wasser direkt in eine Reaktivdestillationskolonne eingespeist und aus dem Produktgemisch e2 in der Reaktivdestillationskolonne ein Produktgemisch f2 im Wesentlichen bestehend aus Isopropanol und Isopropylacetat und Wasser und ein Strom f3 im Wesentlichen bestehend aus Essigsäure gewonnen und der Strom f3 in die Esterbildungszone zurückgeführt;
G) das Produktgemisch f2 wird in einer Destillationskolonne in einen Strom g1 im Wesentlichen bestehend aus Isopropanol und Isopropylacetat und Wasser und einen Strom g2 im Wesentlichen bestehend aus Isopropanol aufgetrennt und der Strom g1 wird in die Reaktivdestillationskolonne zurückgeführt.

2. Verfahren nach Anspruch 1, **dadurch gekennzeichnet, dass** die Dehydrierung in Schritt B) in Gegenwart von Sauerstoff durchgeführt wird.

3. Verfahren nach Anspruch 1, **dadurch gekennzeichnet, dass** die Dehydrierung in Gegenwart von Wasserdampf durchgeführt wird.

4. Verfahren nach Anspruch 1, **dadurch gekennzeichnet, dass** in Schritt F) Wasser in stöchiometrischem Unterschuss eingesetzt wird.

5. Verfahren zur Herstellung von Isopropanol aus Propan mit den Schritten:
A) ein Propan enthaltender Einsatzgasstrom a wird bereitgestellt;
B) der Propan enthaltende Einsatzgasstrom a wird in eine Dehydrierzone eingespeist und Propan wird einer Dehydrierung zum Propen unterworfen, wobei ein Produktgasstrom b enthaltend Propen, nicht umgesetztes Propan, gegebenenfalls Hochsieder, Wasserdampf, Wasserstoff und Leichtsieder erhalten wird;
C) der Produktgasstrom b wird zumindest komprimiert, optional wird der Produktgasstrom b in eine wässrige Phase c1, eine Propen, Propan und gegebenenfalls Hochsieder enthaltende Phase c2 und eine Gasphase c3 enthaltend Wasserstoff und Leichtsieder aufgetrennt;
D) der Produktgasstrom b beziehungsweise die Propen und Propan enthaltende Phase c2 wird in einer Esterbildungszone mit Essigsäure umgesetzt, wobei ein Produktgemisch d enthaltend Isopropylacetat erhalten wird;
E) von dem Produktgemisch d wird ein Propan enthaltender Gasstrom e1 abgetrennt, der gegebenenfalls in die Dehydrierzone zurückgeführt wird, und ein Isopropylacetat enthaltendes Produktgemisch e2 gewonnen;
F) das Isopropylacetat enthaltende Produktgemisch e2 wird in einem Esterspaltungs-Vorreaktor mit Wasser zu einem Produktgemisch f enthaltend Isopropanol, Essigsäure, Isopropylacetat und Wasser umgesetzt;
G) aus dem Produktgemisch f wird in einer Reaktivdestillationskolonne ein Produktgemisch f2 aus Isopropanol, Isopropylacetat und Wasser und ein Strom f3 aus Essigsäure, der in die Esterbildungszone zurückgeführt wird, gewonnen und das Produktgemisch f2 wird in einer nachgeschalteten weiteren Destillationskolonne in einen Strom g1 im Wesentlichen bestehend aus Isopropanol, Isopropylacetat und Wasser und einen Strom g2 aus dem Isopropanol aufgetrennt, wobei der Strom g1 in die Esterspaltungszone zurückgeführt wird.

6. Verfahren nach Anspruch 1, **dadurch gekennzeichnet, dass** die Dehydrierung in Schritt B) in Gegenwart von Sauerstoff durchgeführt wird.

7. Verfahren nach Anspruch 1, **dadurch gekennzeichnet, dass** die Dehydrierung in Gegenwart von Wasserdampf durchgeführt wird.

8. Verfahren nach Anspruch 1, **dadurch gekennzeichnet, dass** Schritt F) in einer Reaktivdestillationskolonne durchgeführt wird.
